# EUROPEAN PATENT SPECIFICATION

(11) **EP 3 914 272 B1**
(45) Date of publication and mention of the grant of the patent: **06.03.2024**
(21) Application number: 20708757.8
(22) Date of filing: 24.01.2020
(51) Int. Cl.: A61K 38/19, A61K 35/407, A61K 38/17, A61K 35/44, A61K 38/18, A61K 38/48, A61P 1/16

(54) **THROMBOPOIETIN MIMETICS FOR MITIGATING LIVER INJURY AND PROMOTING LIVER HYPERTROPHY, REGENERATION AND CELL ENGRAFTMENT IN CONJUNCTION WITH RADIATION AND/OR RADIOMIMETIC TREATMENTS**
THROMBOPOIETIN-MIMETIKA ZUR LINDERUNG EINER LEBERVERLETZUNG UND ZUR FÖRDERUNG DER LEBERHYPERTROPHIE, -REGENERATION UND ZELL-ENGRAFTMENT IN VERBINDUNG MIT BESTRAHLUNGS- UND/ODER RADIOMIMETISCHEN BEHANDLUNGEN
MIMÉTIQUES DE LA THROMBOPOÏÉTINE ET LEUR UTILISATION DANS MÉTHODES D'ATTÉNUATION D'UNE LÉSION HÉPATIQUE ET DE PROMOTION D'UNE HYPERTROPHIE HÉPATIQUE, DE RÉGÉNÉRATION DU FOIE ET DE PRISE DE GREFFE DE CELLULES HÉPATIQUES CONJOINTEMENT AVEC DES TRAITEMENTS PAR RADIOTHÉRAPIE ET/OU RADIOMIMÉTIQUES

(30) Priority: 25.01.2019 US 201962796806 P
(43) Date of publication of application: 01.12.2021
(73) Proprietor: Janssen Pharmaceutica NV, 2340 Beerse (BE); Montefiore Medical Center, Bronx, NY 10467-2401 (US)
(72) Inventor: EICHENBAUM, Gary, 2340 Beerse (BE); GUHA, Chandan, Bronx, NY 10467-2401 (US)
(74) Representative: Høiberg P/S
(86) International application number: PCT/US2020/014934
(87) International publication number: WO 2020/154585

(56) References cited:
- WO-A1-2016/120311
- WO-A2-02/067870
- WO-A2-2008/070583
- YAMAGUCHI MASARU ET AL: "The thrombopoietin mimetic romiplostim leads to the complete rescue of mice exposed to lethal ionizing radiation", SCIENTIFIC REPORTS, , vol. 8 13 July 2018 (2018-07-13), pages 1-12, XP009513711, ISSN: 2045-2322, DOI: 10.1038/S41598-018-29013-5 Retrieved from the Internet: URL:http://www.nature.com/srep [retrieved on 2020-05-11]
- VENU GOPALA REDDY GANGIREDDY ET AL: "Romiplostim in the Management of Thrombocytopenia in a Patient with Autoimmune Hepatitis", DIGESTIVE DISEASES AND SCIENCES, KLUWER ACADEMIC PUBLISHERS-PLENUM PUBLISHERS, NE, vol. 57, no. 9, 25 July 2012 (2012-07-25), pages 2466-2472, XP035104112, ISSN: 1573-2568, DOI: 10.1007/S10620-012-2302-5

## Description

### FIELD OF THE INVENTION

The present disclosure relates to methods for mitigating liver injury, and promoting liver regeneration, hypertrophy and engraftment of liver cells in a subject in need thereof. In particular, this invention relates to methods comprising administering to the subject an effective amount of a thrombopoietin (TPO) mimetic alone or in combination with cell transplant in conjunction with radiation or radiomimetics to promote beneficial effects.

### BACKGROUND OF THE INVENTION

Liver disease is a significant cause of morbidity and mortality world-wide (Yang, J., Int. J. Environ. Res. Public Health 2018, 15, 170). Disease of the liver can be caused by a variety of factors including viral infection, alcohol, and genetic mutations.

Liver tumors occur with much higher frequency in patients with liver disease and are currently on the rise in the US and represent one of the most common malignancies worldwide with an incidence of about 1 million cases annually (Howlader et al., SEER Cancer Statistics Review 1975-2014, 2017:2012-2014). Among liver tumors, hepatocellular carcinoma (HCC) is the second leading cause of cancer-related deaths worldwide, accounting for approximately 745,000 cases per year (Siegel et al., CA Cancer J. Clin., 2015, 65:5-29). Without definitive treatment, liver cancers usually progress and cause significant morbidity and mortality of cancer patients; the 5-year survival of patients with localized liver cancer based is 27.7% (Howlader N, Noone AM, Krapcho M, et al (eds). SEER Cancer Statistics Review, 1975-2009 [Vintage 2009 Populations], National Cancer Institute. Bethesda, MD). Only 15-20% of patients with HCC present with resectable tumors with 5-year overall survival (OS) of 40-70% and 5-year progression-free survival (PFS) of 23-50% (Bismuth H et. al. Semin Liver Dis. 1999, 19(3):311-22).

Current treatment options for HCC patients include liver transplantation, tumor resection, hepatectomy, sorafenib chemotherapy, radiofrequency ablation, trans-arterial chemo/radioembolization, or radiation therapy (Bruix, J. Gastroenterology, 2016, 150: 835-853; Keane, F. Liver Cancer, 2016, 5:198-209). A considerable number of the HCC patients, who cannot get a liver transplant, are also not candidates for these other treatment options; this is due to their much higher risk for mortality from the procedures themselves because of their poor liver function. Portal vein embolization/ligation is a surgical technique that has been developed and applied in recent years to induce liver regeneration through hypertrophy and enhance a patient's healthy liver capacity, thereby making them eligible for these interventions (Cieslak, K. Surgery, 2017, 162: 37-47).

There is a great clinical need for cell transplantation therapies which can restore an injured or diseased liver to health and reestablish its biological functions. For instance, with respect to liver disease, more than 40,000 patients die of terminal liver diseases every year in the United States alone, and it is estimated that approximately 20 million suffer from liver diseases (Hagmann, M., Science, 287:1185, 1187 (2000)). For those with inherited metabolic liver diseases or terminal liver failure, orthotropic liver transplantation (OLT) is the only treatment option, but most die without OLT because of a critical shortage of donors. In theory, many patients with primary or metastatic cancers in the liver could also be cured, or have their survival and/or the quality of life significantly improved, by total hepatectomy with OLT. In practice, however, cancer patients are rarely considered for OLT because of the long waiting lists for donated livers.

Although radiation therapy (RT) is used in one-third of all non-liver cancer patients, the role of RT in hepatic malignancy has traditionally been limited by the low radiation tolerance of the liver. Early studies demonstrated that whole liver radiation in excess of 30-35 Gy is associated with a high risk of radiation induced liver disease (RILD) (Lawrence etal., Int. J. Radiat. Oncol. Biol. Phys., 1995, 31:1237-48; Reed et al., Am. J. Pathol., 1966, 48:597-611). A potential reason for the limited application of liver irradiation therapy is that there are no widely-accepted therapies or radiomitigators currently available for prevention or treatment of RILD. Further, the probability of RILD increases with baseline liver dysfunction (Pan et al., Int. J. Radiat. Oncol. Biol. Phys., 2010, 76:S94-100). Recently a retrospective study reported 11.2% of patients undergoing stereotactic body radiation therapy (SBRT) to the liver developed RILD (Lo et al., PLoS ONE, 2017, 12(5): e0177793). Patients receiving preparative irradiation for bone marrow transplant who also developed RILD experienced significant mortality (84%) (Fulgenzi et al., Hepatic Med. Evid. Res., 2016, 8:105-113). Furthermore, the liver is one of the organs that are commonly coincidentally irradiated during RT treatment of gastrointestinal cancers because of its proximity to the gastrointestinal tract and its large size (Pan et al., Int. J. Radiat. Oncol. Biol. Phys., 2010, 76:S94-100).

The liver is one of the most radio-sensitive organs (Emami et al., Int. J. Radiat. Oncol. Biol. Phys., 1991, 21:109-22) due to its high vascularity and the extreme radio-sensitivity of the endothelial cells lining its vessels (Baker et al., Cancer Invest., 1989, 7:287-94). With fixed physiologic organ size, non-irradiated liver undergoes hypertrophy to compensate for the atrophy of the irradiated lobe (Guha et al., Seminars in Oncology, 2011, 21(4):256-263). Hepatic irradiation may also cause injury to liver sinusoidal endothelial cells (LSEC), which can result in sinusoidal congestion, edematous widening of the sub-endothelial space of central and sub-lobular veins, and even sinusoidal obstruction (Pan et al., Int. J. Radiat. Oncol. Biol. Phys., 2010, 76:S94-100). As a result, careful treatment planning is required to minimize irradiated tissue to protect the patient and prevent RILD (Kalman et al., Int. J. Radiat. Oncol., 2017, 98:662-682; Guha et al., Seminars in Oncology, 2011, 21(4):256-263). With advanced treatment planning, very high doses (up to 90-100GY) can be administered if the radiation volume is small enough (~1/3 of the total liver volume)( Dawson et al., Int. J. Radiat. Oncol. Biol. Physics., 2002, 53:810-821). Stereotactic Body Radiation Therapy (SBRT) and administration of radiation over multiple instances (fractionation) are other approaches that are being applied to reduce the overall toxicity profile of radiation and enabling its use in patients.

Thrombopoietin (TPO) is a growth factor that is synthesized and secreted by the liver. In addition to acting as a humoral growth factor that stimulates the proliferation and differentiation of megakaryocytes through the thrombopoietin receptor (TPO-R or c-Mpl), recombinant human TPO (rhTPO) has been shown to promote platelet activation and liver endothelial cell growth and migration in vitro (Cardier et al., Blood, 1998, 91:923-929).

Defibrotide is the only clinically-approved drug for mitigation of sinusoidal obstruction syndrome associated with radiation-induced liver disease (RILD). Defibrotide is a mixture of nucleotides purified from porcine intestinal mucosa DNA (Fulgenzi et al., Hepatic Med. Evid. Res., 2016, 8:105-113; Guha etal., Seminars in Oncology, 2011, 21(4):256-263) and it reduces sinusoidal injury by modulating the activity of LSECs, which reduces sinusoidal permeability and LSEC dehiscence.

There are also no clinically-approved drugs for promoting engraftment of liver cells, such as sinusoidal endothelial cells, stem cells, hepatocyte progenitor cells or hepatocytes in the liver. In addition, there are also no drugs approved for enhancing liver hypertrophy.

Yamaguchi Masaru et al., Scientific Reports, 2018, vol. 8: 1-12 discloses a role of romiplostim in the prevention/protection of bone marrow damage associated with radiotherapy.

Venu Gopala Reddy Gangireddy et al, Digestive Diseases and Sciences, 2012, vol. 57(9): 2466-2472 relates to the use of Romiplostin in the management of thrombocytopenia in autoimmune hepatitis.

WO 02/067870 and WO 2016/120311 relate to different compounds for use in the treatment of liver conditions induced by radiation therapy.

### BRIEF SUMMARY OF THE INVENTION

It is now discovered that thrombopoietin (TPO) mimetics can mitigate liver injury, promote liver regeneration, increase the volume of liver, and promote engraftment of liver cells in a subject in need thereof. For example, it is found that TPO mimetics have significant mitigating effects on targeted radiation therapy-induced liver diseases (RILDs), can enhance the hypertrophy of the non-irradiated lobe of the liver and promote engraftment of endogenous marrow derived or exogenous liver cells, such as liver sinusoidal endothelial cells (LSECs), in a subject in need thereof, when used alone or together with the administration of liver cells, such as LSECs, and/or a protein factor. It is also discovered that treatment with a TPO mimetic prior to radiation can have an enhanced effect on protection and repair of liver compared to a TPO mimetic treatment after the radiation. Whereas a LSEC transplant is required to protect against RILD when TPO mimetic is given post-irradiation, no LSEC transplant is required to protect against RILD when TPO mimetic is given prophylactically prior to irradiation.

Accordingly, in one general aspect, the application relates to a method of mitigating RILD in a subject in need thereof, the method comprising: administering to the subject an effective amount of a thrombopoietin (TPO) mimetic comprising the amino acid sequence of SEQ ID NO: 1, more preferably the TPO mimetic is RWJ-800088 or romiplostim. Examples of the RILD include, but are not limited to, hepatomegaly, ascites, elevated liver enzymes, thrombocytopenia, hepatic sinusoidal obstruction syndrome (SOS), hepatic central venous occlusive disease (VOD), and hepatic fibrosis.

In certain embodiments, the TPO mimetic is administered to the subject in combination with another active agent. For example, the TPO mimetic can be administered in combination with transplantation of liver cells, such as LSECs, hepatocytes, progenitor cells, pluripotent stem cells, hepatic stem cells, or recombinant liver cells expressing one or more transgenes. The TPO mimetic can also be administered in combination with a protein factor, such as a bone marrow stem cell mobilizing factor such as a CXCR4 antagonist; a growth factor, such as GM-CSF, GCSF, or FLT3 ligand; or optionally further with hepatocyte growth factor. The TPO mimetic can further be administered in combination with transplantation of liver cells (such as LSECs) and administration of a growth factor. The TPO mimetic can be administered to the subject before, after, or simultaneously with the other active agents, such as liver cells (e.g., LSECs, hepatocytes) or protein factors.

In certain embodiments, the subject in need of a treatment of the application is a subject treated with a radiation therapy, preferably a targeted radiation therapy, which may result in RILD, such as a targeted radiation therapy for a liver disease, preparative irradiation for bone marrow transplant, or targeted radiation therapy for a gastrointestinal cancer. In another embodiment, the subject is treated with a preparative hepatic irradiation (HIR) for engraftment of liver cells. The TPO mimetic can be administered to the subject before, after, or simultaneously with the radiation therapy. In certain embodiments, the TPO mimetic is administered to the subject at least about 24 hours before to at least about 24 hours after the subject is administered with a dose of radiation. In some embodiments, the TPO mimetic is administered 24 to 2 hours before or after a dose of radiation. In other embodiments, the TPO mimetic is administered 1 minute to 2 hours before or after a dose of radiation. In some embodiments, the TPOm is administered 2 to 24 hours after a radiation dose, for example, together with the administration of liver cells.

In certain embodiments, the subject is treated with targeted radiation, preferably to the liver, at a dose of 10-70 Gray (Gy) in 1 to 10 fractions.

In certain embodiments, the effective amount of the TPO mimetic is about 1 to about 5 µg/kg of body weight of the subject. In preferred embodiments, the effective amount of the TPO mimetic is about 1 µg/kg of body weight of the subject. In certain preferred embodiments, the effective amount of the TPO mimetic is about 3 µg/kg of body weight of the subject when administered subcutaneously or intravenously.

In certain embodiments, the effective amount of the TPO mimetic is administered to the subject by intravenous, intramuscular, or subcutaneous injection. In preferred embodiments, the TPO mimetic is administered by subcutaneous injection.

The invention is defined by the claims. Any subject matter falling outside the scope of the claims is provided for information purposes only. The references to methods of treatment in this description are to be interpreted as references to the compounds, pharmaceutical compositions and medicaments of the present invention for use in a method for the treatment of the human (or animal) body by therapy (or for diagnosis)

### BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

The patent or application file contains at least one drawing executed in color. Copies of this patent or patent application publication with color drawings will be provided by the Office upon request and payment of the necessary fess.

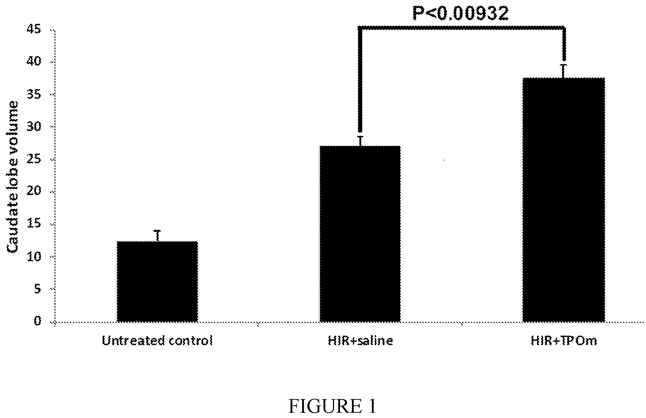

Figure 1 shows mean volume of untreated caudate lobe following partial hepatic irradiation (HIR) with and without TPOm (RWJ-800088) administration.

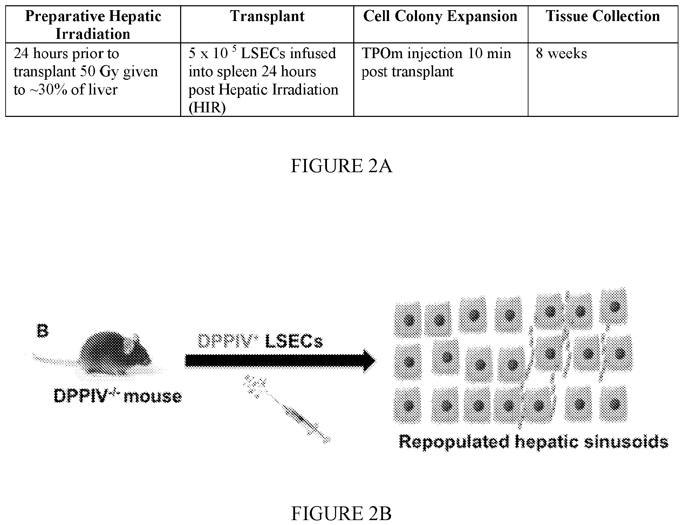

Figure 2A illustrates the time line for the study of targeted hepatic liver irradiation (HIR) + TPOm (RWJ-800088) + LSEC transplantation in Experiment 2. Figure 2B is a graphic representation of the mouse DPPIV (-/-) model transplanted with DPPIV (+/+) LSEC cells, used for the detection of the repopulation of transplanted cells in the hepatic sinusoids.

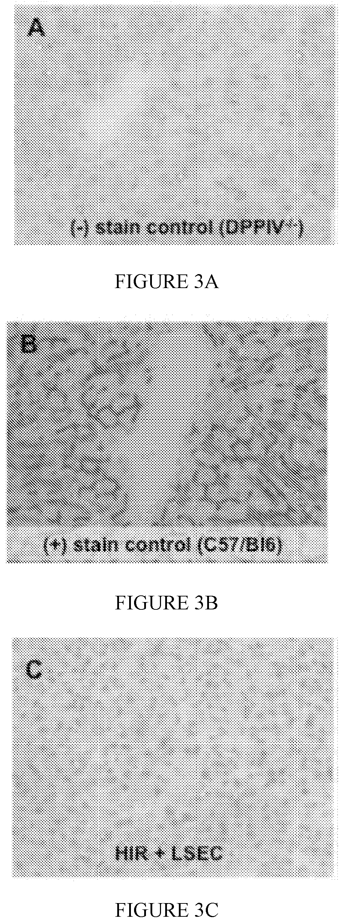

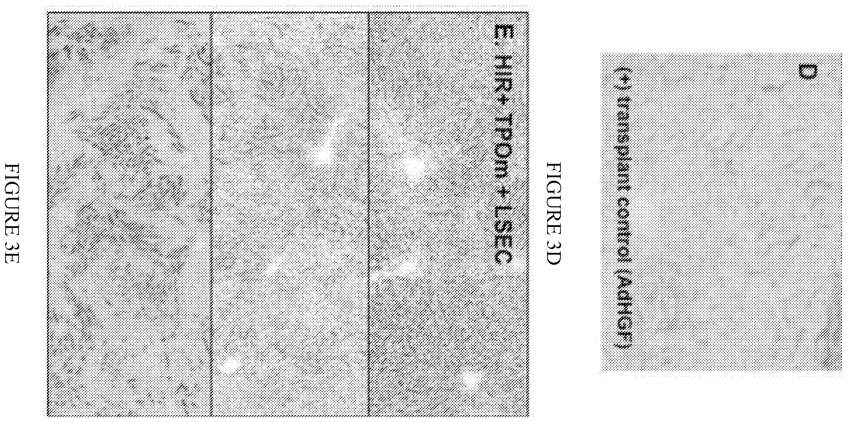

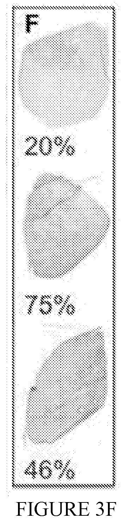

Figures 3A-3F show immunohistochemical staining to detect the DPPIV (+/+) LSEC cells following transplantation that repopulate the irradiated liver of animals receiving TPOm (RWJ-800088): Figure 3A shows the DPPIV (-/-) transplant recipient liver before transplantation showing no DDPIV staining (negative control); Figure 3B shows positive DPPIV staining in LESC donor C57B16 mice that are DPPIV(+/+) (positive control); Figure 3C shows HIR + LSEC without DPPIV (+/+) cell repopulate; Figure 3D shows LSECs + AdHGF treatment and DPPIV(+/+) cells repopulating the liver; Figure 3E shows that massive LSEC repopulation is seen when TPOm is given following hepatic irradiation; and Figure 3F shows the repopulated liver lobes in low power.

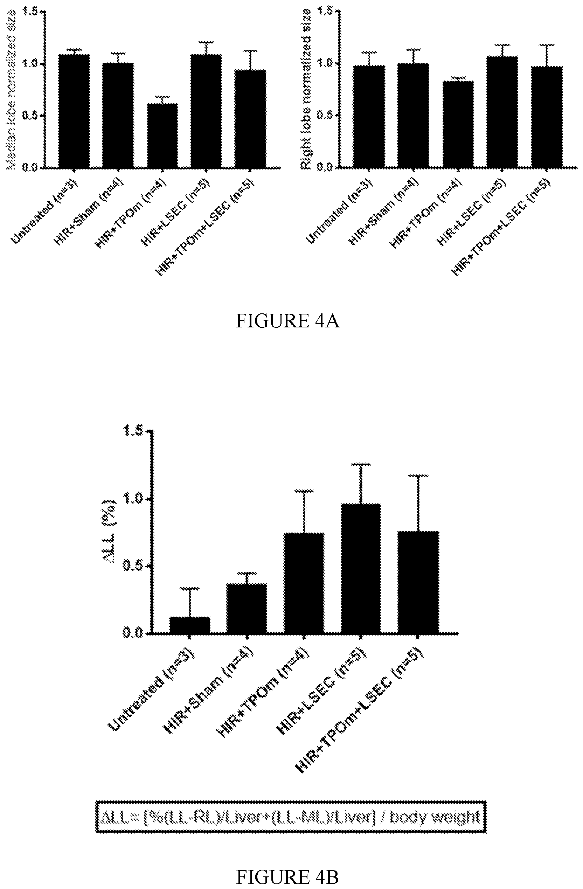

Figures 4A-4B demonstrate that treatments with TPOm (RWJ-800088) reduce liver injury in irradiated tissue: Figure 4A shows relative change of the irradiated lobe, and Figure 4B shows relative change in non-irradiated lobe.

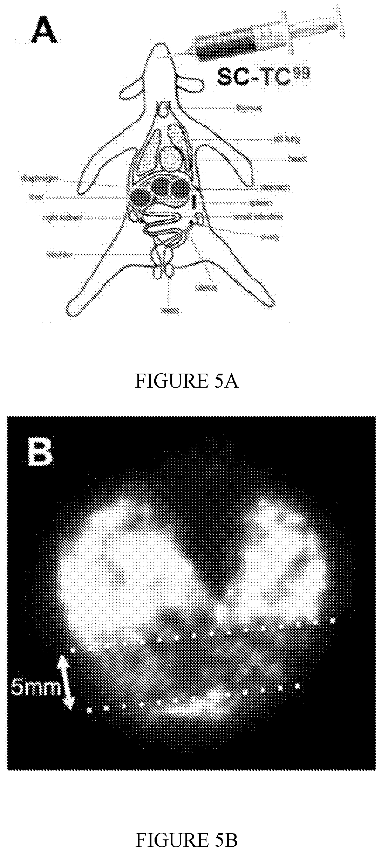

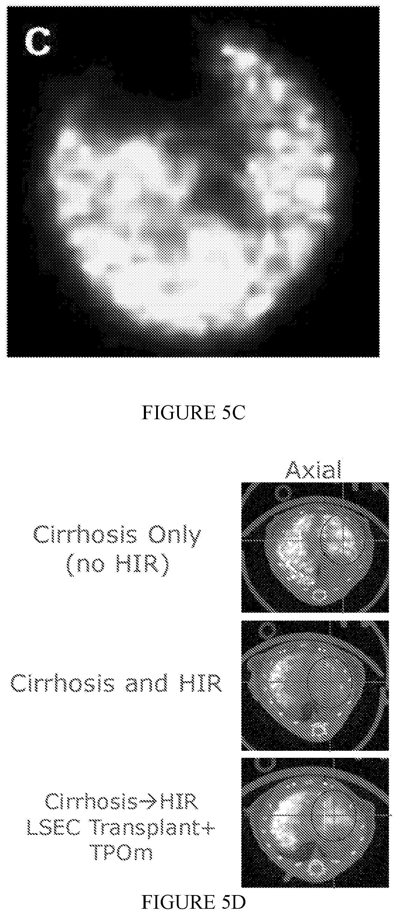

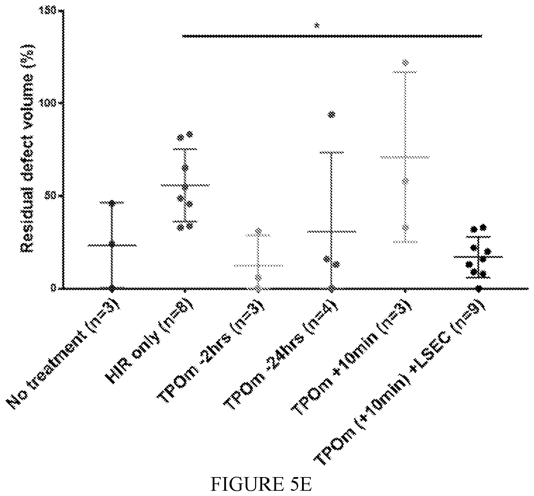

Figures 5A-5E show that SPECT-CT quantifies residual perfusion deficiency in the liver 2 months post transplantation: Figure 5A demonstrates basic schematic of SPECT principle; Figure 5B shows perfusion deficiency in irradiated liver tissue corresponding to the 5 mm collimator used for radiation administration; Figure 5C shows perfusion recovery in animals treated with LSEC transplant followed by TPOm (RWJ-800088) injection; Figure 5D shows that residual defect 2 months post radiation was significantly reduced in TPOm + LSEC group in a cirrhotic mouse liver; and Figure 5E shows the relative percentage of defect volume of irradiated liver tissue compared to non-irradiated tissue following administration of TPOm at 2 hours and 24 hours prior to Hepatic Irradiation (HIR) and 10 minutes post HIR without a LSEC transplant, compared to following an LSEC transplant 24 hours post HIR and administration of TPOm 24 hours and 10 minutes post HIR. The data shows significant mitigation of defect formation when TPOm is administered with LSEC transplant post irradiation and significant reduction in defect volume with TPOm alone when it is administered without an LSEC transplant.

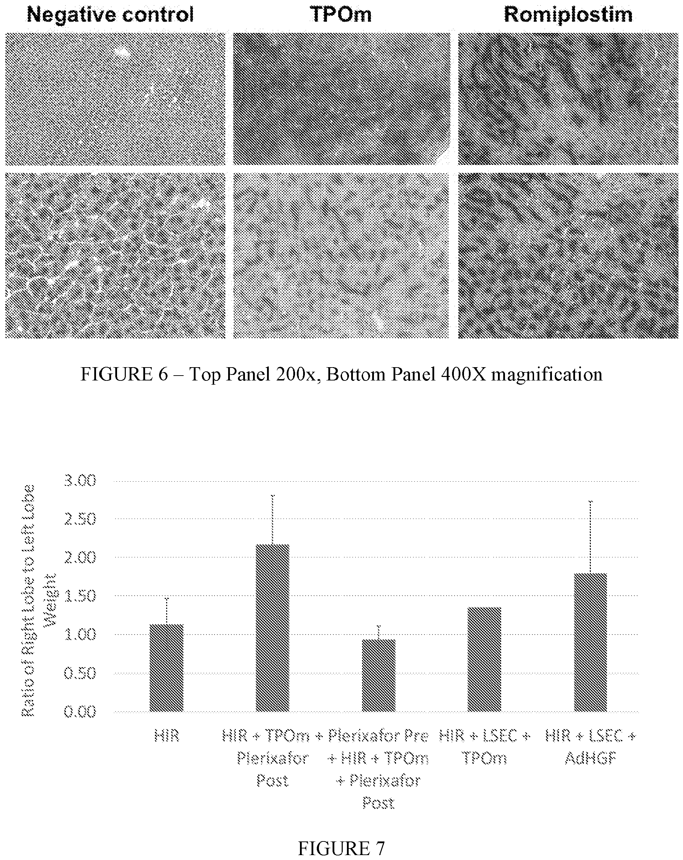

Figure 6 shows engraftment of LSECs when TPOm or Romiplostim are administered 10 minutes following LSEC transplantation 4-5 days post HIR.
Figure 7 shows ratio of the weight of the non-irradiated right lobe to the weight of the irradiated left lobe in the study to evaluate the effect of TPOm + a transplant of liver sinusoidal endothelial cells (LSEC), TPOm + plerixafor administered post irradiation and TPOm + Plerixafor administered pre- and post- irradiation on radiation induced liver disease.

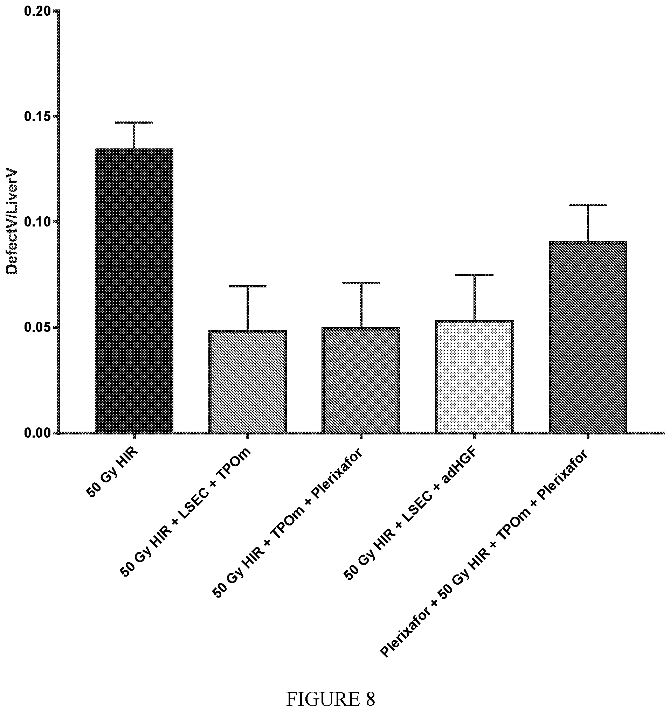

Figure 8 shows defect volume/liver volume in cirrhotic mice quantified by SPECT-CT in the study to evaluate the effect of TPOm + a transplant of liver sinusoidal endothelial cells (LSEC), TPOm + plerixafor administered post irradiation and TPOm + Plerixafor administered pre- and post- irradiation on radiation induced liver disease.

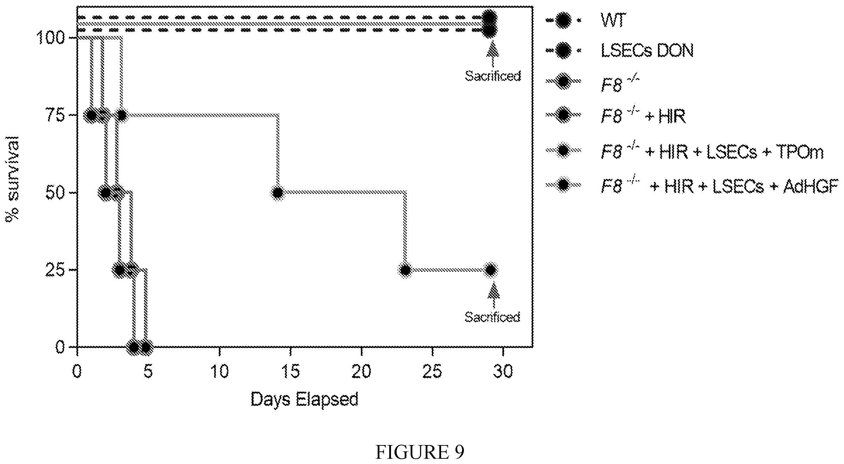

Figure 9 presents a Kaplan-Meyer curve showing the survival post-the Drabkin's bleeding test on Day 120 in Experiment 6. The Days Elapsed are days post the Day 120 bleeding test.

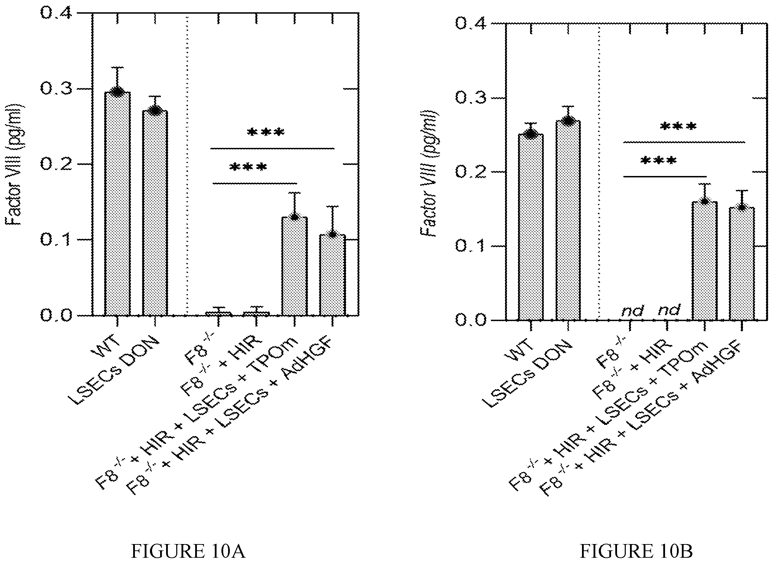

Figures 10A and 10B demonstrate ELISA analysis of production of FVIII in the treated HIR+ LSECs + JNJ-26366821 or AdHGF groups compared to non-treated Haemophilia A mice for plasma (10A) or for liver (10B). n = 5 and ***P < 0.005. WT; wild type, HIR ; Hepatic Irradiation; LSECs ; Liver sinusoidal endothelial cells.

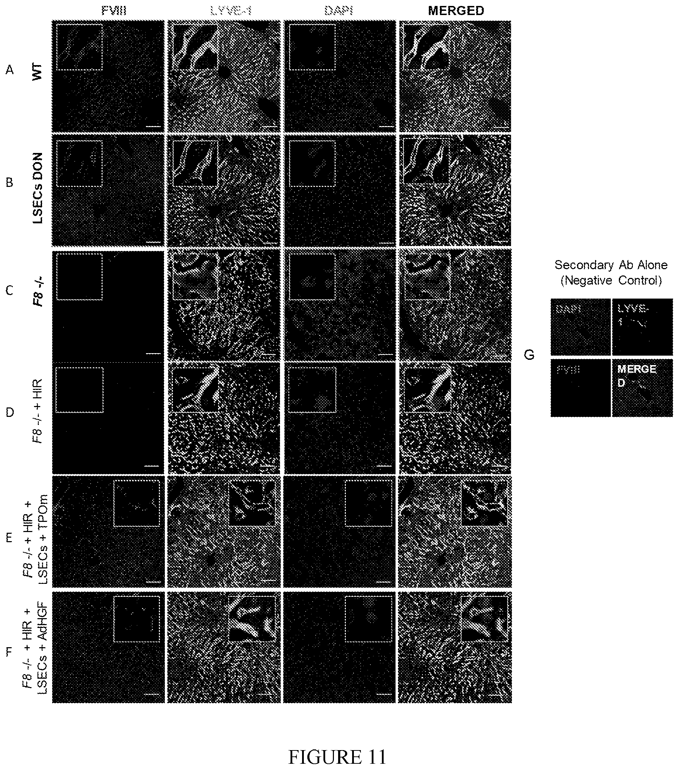

Figure 11 shows immunofluorescence evaluating liver slides stained for Factor VIII (red), LYVE-1 (green) - selective antibody for liver sinusoidal endothelial cells, DAPI (blue) -DNA stain showing cells, and merged areas for FVII + LYVE-1 (yellow). (A) WT = C57/BL6, (B) LSEC Donor mice = B6129SF2/J, (C) Hemophilia A mice F8(-/-), (D) Hemophilia A mice F8(-/-) + HIR, (E) F8(-/-) + HIR + LSECs + JNJ-26366821 (TPOm), (F) F8(-/-) + HIR + LSECs + Ad HGF and (G) Secondary antibody negative control immunofluorescence. Full image = 10x and zoom image = 64x magnification and white bars are 20 µm (micrometer).

### DETAILED DESCRIPTION OF THE INVENTION

This disclosure is based, at least in part, on the identification of a thrombopoietin (TPO) mimetic as a therapeutic for mitigating a radiation-induced liver disease in a subject in need thereof. The TPO mimetic can be formulated and administered to the subject who is or will be exposed to the radiation therapy to mitigate the radiation-induced liver disease.

Various publications, articles and patents are cited or described in the background and throughout the specification. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes ± 10% of the recited value. For example, a concentration of 1 mg/mL includes 0.9 mg/mL to 1.1 mg/mL. Likewise, a concentration range of 1% to 10% (w/v) includes 0.9% (w/v) to 11% (w/v). As used herein, the use of a numerical range expressly includes all possible subranges, all individual numerical values within that range, including integers within such ranges and fractions of the values unless the context clearly indicates otherwise.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the invention.

As used herein, the terms "comprises," "comprising," "includes," "including," "has," "having," "contains" or "containing," or any other variation thereof, will be understood to imply the inclusion of a stated integer or group of integers but not the exclusion of any other integer or group of integers and are intended to be non-exclusive or open-ended. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

As used herein, the term "consists of," or variations such as "consist of' or "consisting of," as used throughout the specification and claims, indicate the inclusion of any recited integer or group of integers, but that no additional integer or group of integers can be added to the specified method, structure, or composition.

As used herein, the term "consists essentially of," or variations such as "consist essentially of' or "consisting essentially of," as used throughout the specification and claims, indicate the inclusion of any recited integer or group of integers, and the optional inclusion of any recited integer or group of integers that do not materially change the basic or novel properties of the specified method, structure or composition. See M.P.E.P. § 2111.03.

As used herein, "subject" means any animal, preferably a mammal, most preferably a human, who will be or has been treated by a method according to an embodiment of the invention. The term "mammal" as used herein, encompasses any mammal. Examples of mammals include, but are not limited to, cows, horses, sheep, pigs, cats, dogs, mice, rats, rabbits, guinea pigs, monkeys, humans, etc., more preferably a human.

The words "right", "left", "lower" and "upper" designate directions in the drawings to which reference is made.

It should also be understood that the terms "about," "approximately," "generally," "substantially" and like terms, used herein when referring to a dimension or characteristic of a component of the preferred invention, indicate that the described dimension/characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

As used herein, the term "in combination", in the context of the administration of two or more therapies to a subject, refers to the use of more than one therapy. The use of the term "in combination" does not restrict the order in which therapies are administered to a subject. For example, a first therapy (e.g., a composition described herein) can be administered prior to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject.

The term "RILD" or "radiation-induced liver disease," as used herein, refers to an acute response during or within the first few weeks of radiation therapy (RT) or as a late-response months after RT. Examples of radiation-induced liver disease (RILD) can include, but are not limited to, hepatomegaly, hepatic necrosis, apoptosis, ascites, elevated liver enzymes, thrombocytopenia, hepatic sinusoidal obstruction syndrome (SOS), hepatic central venous occlusive disease (VOD), and hepatic fibrosis.

RILD is one of the complications of RT. Although RILD typically occurs 4-8 weeks after termination of RT, it has been reported to appear as early as 2 weeks or as late as 7 months after RT (Guha et al., Seminars in Oncology, 2011, 21(4):256-263; Khozouz et al., J. Clin. Oncol., 2008, 26(29):4844-4845). There are two types of RILD: classic RILD and non-classic RILD. Patients with classic RILD usually have symptoms of fatigue, abdominal pain, increased abdominal girth, hepatomegaly and anicteric ascites 1-3 months after liver RT (Lawrence et al., Int. J. Radiat. Oncol. Biol. Phys., 1995, 31:1237-48). In addition, the level of alkaline phosphatase (ALP) increases by more than twofold that of normal levels, whereas levels of transaminase and bilirubin remain normal (Liang et al., Radiother. Oncol., 2011, 98(2):265-9). A pathological hallmark of classic RILD is hepatic veno-occlusive disease (VOD), which is characterized by complete obliteration of the central vein lumina by erythrocytes trapped in a network of reticulin and collagen fibers (Reed et al., Am. J. Pathol., 1966, 48:597-611; Ogata et al., Tokushima J. Exp. Med., 1963, 10: 240-251). The trapped erythrocytes create vascular congestion, leading to decreased oxygen delivery to the central zone. This hypoxic environment results in both the death of centrilobular hepatocytes and atrophy of the inner hepatic plate, leading to hepatic dysfunction. In addition, hepatic stellate cell activation contributing to hepatic fibrosis is a common characteristic in patients with classic RILD (Sempoux et al., Hepatology, 1997, 26(1):128-34). Patients who develop non-classic RILD have underlying chronic hepatic diseases, such as cirrhosis and viral hepatitis, and show more dysregulated hepatic functions with jaundice and/or remarkably elevated serum transaminases (a more than fivefold increase compared to normal levels) rather than ALP (Pan et al., Int. J. Radiat. Oncol. Biol. Phys., 2010, 76:S94-100; Cheng et al., Int. J. Radiat. Oncol. Biol. Phys., 2004, 60(5):1502-9). For example, hepatocellular loss, hepatic dysfunction, hepatic sinusoidal endothelial death and HSC activation have been detected in non-classic RILD.

As used herein, "SOS" or "sinusoidal obstruction syndrome" refers to a veno-occlusive disease (VOD) of the liver. The SOS is a distinctive and potentially fatal form of hepatic injury that occurs predominantly after exposure to a radiomimetic agent, radiation, or transplantation. SOS can present in an acute, subacute or chronic form usually with abdominal pain and swelling, with evidence of portal hypertension and variable degrees of serum enzyme elevations and jaundice. Liver histology demonstrates obstruction of sinusoids in central areas with hepatocyte necrosis and hemorrhage.

### Radiation Therapy

The term "TRT" or "targeted radiation therapy", as used herein, refers to a therapy using ionizing radiation, or a radiomimetic agent, that is preferentially targeted or localized to a specific organ or part of the body. It is generally used as part of cancer treatment. TRT, such as targeted ionizing radiation therapy, is sometimes also referred to as radiation treatment, radiotherapy, irradiation, or x-ray therapy. There are three main divisions of targeted ionizing radiation therapy: external beam radiation therapy (EBRT or XRT), internal radiation therapy, and systemic radioisotope therapy. Sometime, the radiation can be given in several treatments to deliver the same or slightly higher dose, which is called fractioned radiation therapy. As used herein, the term "radiomimetic agent" or "radiomimetic chemical agent" refers to a chemical agent that produces an effect similar to that of ionizing radiation when administered to a subject. Examples of such effect include DNA damage. Examples of radiomimetic chemical agents include, but should not be considered limited to, etoposide, doxorubicin, carboplatin, and bleomycin. Radiomimetic chemical agents such as those described herein can be administered locally to a subject to allow for a targeted application of the agent in a therapeutic manner.

External beam radiation therapy (EBRT) uses a machine that directs high-energy rays from outside the body into the tumor. Current radiation technology allows the precise delivery of external beam radiation therapy, such as targeted radiation therapy which uses computers to create a 3-dimensional picture of the tumor in order to target the tumor as accurately as possible and give it the highest possible dose of radiation while sparing normal tissue as much as possible. Examples of EBRT include, but are not limited to, stereotactic radiation therapy, image guided radiation therapy (IGRT), intensity modulated radiation therapy (IMRT), helical-tomotherapy, proton beam radiation therapy, and intraoperative radiation therapy (IORT). Among them, stereotactic radiation is a specialized type of external beam radiation therapy. It uses focused radiation beams targeting a well-defined tumor using extremely detailed imaging scans. There are two types of stereotactic radiation: stereotactic radiosurgery (SRS) is for stereotactic radiation treatment of the brain or spine, while stereotactic body radiation therapy (SBRT) refers to more precise targeted radiation treatment to organs within the body, such as the lungs and livers.

Internal radiation is also called brachytherapy, in which a radioactive implant is put inside the body in or near the tumor. It allows a higher dose of radiation in a smaller area than might be possible with external radiation treatment. It uses a radiation source that's usually sealed in a small holder called an implant. Different types of implants may be called pellets, seeds, ribbons, wires, needles, capsules, balloons, or tubes. Several such examples of internal radiation are Y-90 SIR-sphere and/or Thera-Sphere.

Transarterial chemoembolization (TACE) involves the use of radiomimetic chemotherapeutics to treat liver cancers.

Targeted systemic radioisotope therapy (SRT) is also called unsealed source radiotherapy. Targeted radioactive drugs are used in SRT to treat certain types of cancer systemically, such as thyroid, bone, and prostate. These drugs, which are typically linked to a targeting entity - such as a monoclonal antibody or a cell-specific ligand, can be given by mouth or put into a vein; they then travel through the body until reaching the desired target, where the drug will accumulate in a relatively high concentration.

TRT may result in various degrees of hepatic decompensation and manifestation of RILD. RILD can be manifested clinically by the development of anicteric ascites. RILD resulting from TRT is undesirable and requires mitigation.

However, targeted irradiation can be used to confer a growth disadvantage on endogenous cells of the target organ, such as cancer cells of a diseased liver, and enhance the growth of engrafted healthy cells. For instance, preparative hepatic irradiation (HIR) can be administered to injure host hepatocytes and prevent them from proliferating and competing with donor hepatocytes in response to mitotic stimuli. Preparative irradiation is routinely used for bone marrow transplantation (Thomas, E. et al., N. Engl. J. Med., 292:832-843 (1975)). Preparative HIR was also used to facilitate hepatocyte transplant in rodent models (see, e.g., Guha, C. et al., Int. J. Radiat. Oncol. Biol. Phys., 49:451-457 (2001); Takahashi, M. et al., Gene. Ther., 10:304-313 (2003)). In humans, radiation-induced liver injury is a function of mean liver radiation dose and the irradiated liver volume (Lawrence, T. et al., Int. J. Radiat. Oncol. Biol. Phys., 19:1041-1047 (1990); Dawson, L. et al., Int. J. Radiat. Oncol. Biol. Phys., 53:810-821 (2002)). A lower dose of HIR, or partial liver irradiation, is desirable for clinical application of HIR. HIR can be safely administered in the clinic using stereotactic radiosurgery (SRS) or 3-D conformal TRT (3-D CRT) techniques. Accordingly, preparative HIR can be used as a preparative regimen for a method of the application.

Partial liver irradiation is well tolerated in cancer patients and with modern techniques of IMRT. Doses higher than 50 Gy to parts of the liver can safely be offered to patients in the clinic. For example, it is reported that HIR administered to the anterior lobes of the liver can induce selective lobar repopulation of donor cells (Deb, N. et al., Hepatology, 34 (4): 153A., 34:153A (2001)). It is accordingly contemplated that partial liver irradiation can be used as a preparative regimen for a method of the application.

As used herein "a subject treated with a targeted radiation therapy" refers to a subject who is undergoing a targeted radiation treatment and the treatment can be before, after or simultaneously with the administration of the TPO mimetic.

### TPO mimetic

As used herein, a "TPOm", "TPO mimetic" or "thrombopoietin mimetic" refers to a compound comprising a peptide capable of binding to and activating a thrombopoietin receptor or c-mpl. Preferably, in a TPO mimetic useful for the invention, the peptide capable of binding to and activating a thrombopoietin receptor has no significant homology with thrombopoietin (TPO). The lack of homology with TPO reduces the potential for generation of antibodies against endogenous TPO. Examples of such peptide useful in a TPO mimetic include, but are not limited to, those described in U.S. Publication Nos. 2003/0158116; 2005/0137133; 2006/0040866; 2006/0210542; 2007/0148091; 2008/0119384; U.S. Patent Nos. 5,869,451; 7,091,311; 7,615,533; 8,227,422; International Patent Publications WO2007/021572; WO2007/094781; and WO2009/148954. More preferably, in a TPO mimetic useful for the invention, the peptide capable of binding to and activating a thrombopoietin receptor is covalently linked to a moiety that improves one or more properties of the peptide. By way of a nonlimiting example, the moiety can be a hydrophilic polymer, including but not limited to polyethylene glycol (PEG), polypropylene glycol, polylactic acid and polyglycolic acid. The moiety can also be a polypeptide, such as a Fc region or an albumin.

The TPO mimetic for use according to the present invention comprises a peptide having the amino acid sequence of: IEGPTLRQXaaLAARYaa (SEQ ID NO:1), wherein Xaa is tryptophan (W) or (β-(2-naphthyl)alanine (referred to herein as "2-Nal"), and Yaa is alanine (A) or sarcosine (referred herein as "Sar"). Preferably, the peptide of SEQ ID NO: 1 is covalently linked to a PEG or fused to a Fc domain.

In some embodiments, a TPO mimetic useful for the invention comprises a peptide of SEQ ID NO: 1 covalently linked to a PEG, preferably a PEG having an average molecular weight of between about 5,000 to about 30,000 daltons. Preferably, the PEG is selected from the group consisting of monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH2), monomethoxypolyethylene glycol-tresylate (MePEG-TRES), and monomethoxypolyethylene glycol-imidazolyl-carbonyl (MePEG-IM). The PEGylation of the peptide leads to a reduced clearance of the compound without loss of potency. See, e.g., U.S. Patent No. 7,576,056.

In one preferred embodiment, the TPO mimetic useful for the invention is RWJ-800088. As used herein, "RWJ-800088" refers to a 29-mer peptide having two identical 14-mers (SEQ ID NO:2) linked by a lysinamide residue as follows: 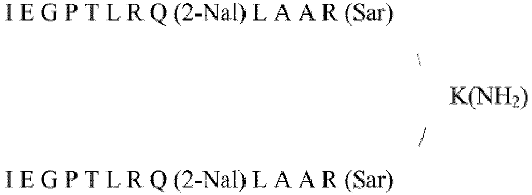 and having a methoxypoly(ethylene glycol) (MPEG) covalently linked to each N-terminal isoleucine, or a pharmaceutically acceptable salt or ester thereof. The RWJ-800088 is thus composed of two 14 amino acid peptide chains of SEQ ID NO: 1, where Xaa is 2-Nal and Yaa is Sar, linked by lysinamide reside, and each N-terminal isoleucine is linked to a methoxy polyethylene glycol (MPEG) chain. Accordingly, RWJ-800088 has an abbreviated molecular structure of (MPEG-Ile-Glu-Gly-Pro-Thr-Leu-Arg-Gln-(2-Nal)-Leu-Ala-Ala-Arg-(Sar))₂-Lys-NH₂; wherein (2-Nal) is β-(2-naphthyl)alanine, (Sar) is sarcosine and MPEG is methoxypoly(ethylene glycol), or a pharmaceutically acceptable salt or ester thereof. Preferably, the MPEG has an approximately 20,000 Dalton molecular weight or represents methoxypolyethylene glycol20000.

In one embodiment, RWJ-800088 has a molecular structure of formula (I), or a pharmaceutically acceptable salt or ester thereof:

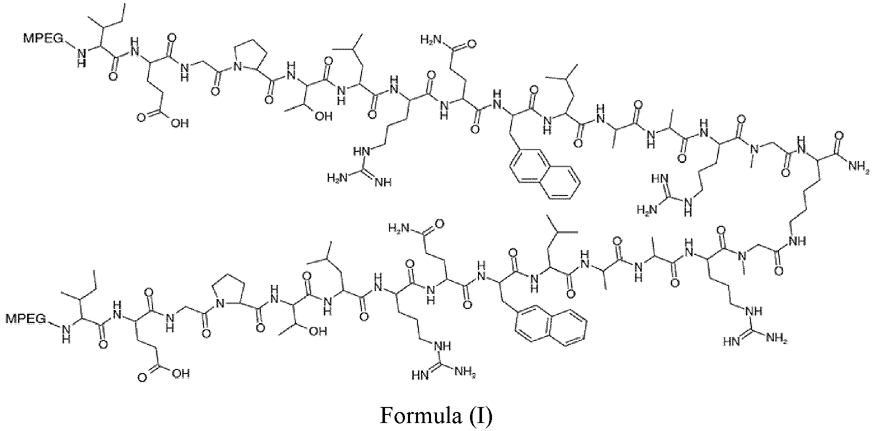

In a preferred embodiment, the MPEG in RWJ-800088 is methoxypolyethyleneglycol20000, and the RWJ-800088 has the full chemical name of: methoxypolyethyleneglycol20000-propionyl-L-Isoleucyl-L-Glutamyl-Glycyl-L-Prolyl-L-Threonyl-L-Leucyl-L-Arginyl-L-Glutaminyl-L-2-Naphthylalanyl-L-Leucyl-L-Alanyl-L-Alanyl-L-Arginyl-Sarcosyl-Ne-(methoxypolyethyleneglycol20000-propionyl-L-Isoleucyl-L-Glutamyl-Glycyl-L-Prolyl-L-Threonyl-L-Leucyl-L-Arginyl-L-Glutaminyl-L-2-Naphthylalanyl-L-Leucyl-L-Alanyl-L-Alanyl-L-Arginyl-Sarcosyl-)-Lysinamide, or a pharmaceutically acceptable salt or ester thereof. The molecular weight of the peptide without PEG is 3,295 Daltons and with two 20,000 Dalton MPEG chains is approximately 43,295 Daltons.

In some embodiments, the TPO mimetic useful for the invention comprises a peptide of SEQ ID NO: 1 fused to a Fc domain. Fusing the peptide to a Fc domain can stabilize the peptide *in vivo.* See, e.g., U.S. Patent No. 6,660,843.

In another preferred embodiment, the TPO mimetic useful for the invention is romiplostim. As used herein, "romiplostim" refers to fusion protein having a Fc domain linked to the N-terminal isoleucine of the peptide of SEQ ID NO: 1, where Xaa is W and Yaa is A. In particular, romiplostim has the following amino acid sequence: 
MDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQ
GNVFSCSVMHEALHNHYTQKSLSLSPGKGGGGGIEGPTLRQWLAARAGG
GGGGGIEGPTLRQWLAARA (SEQ ID NO:4),
 It has the thrombopoietin receptor binding domain amino acid sequence of IEGPTLRQWLAARA (SEQ ID NO:3).

### Dosage and Administration

In the current invention, the inventors discovered that TPO mimetics have significant mitigating effects on RILDs, can enhance the hypertrophy of the non-irradiated lobe of the liver and promote engraftment of liver cells, such as liver sinusoidal endothelial cells (LSECs). Thus, methods of the invention comprise administering to a subject in need thereof an effective amount of the TPO mimetic as disclosed herein to thereby achieve one or more beneficial results, such as mitigating one or more RILDs, promoting liver cell engraftment and enhancing hypertrophy of a non-irradiated lobe of liver, in the subject in need thereof, such as a subject treated with a radiation therapy, or a subject in need of liver cell transplantation.

The TPO mimetic can, for example, be administered as an active ingredient of a pharmaceutical composition in association with a pharmaceutical carrier or diluent. The TPO mimetics can be administered by oral, pulmonary, parental (intramuscular (IM), intraperitoneal (IP), intravenous (IV) or subcutaneous (SC) injection), inhalation (via a fine powder formulation), transdermal, nasal, vaginal, rectal, or sublingual routes of administration can be formulated in dosage forms appropriate for each route of administration. Preferably, the TPO mimetic is administered by subcutaneous injection. For example, International Publication No. WO 1993/25221 (Bernstein et al.) discloses biodegradable polymer microspheres containing erythropoietin (EPO), which can be administered topically, locally or systemically by parenteral administration or enteral administration, preferably oral administration. WO 1994/17784 (Pitt et al.) discloses that EPO can be administered systemically via pulmonary route and that such delivery results in comparable levels of therapeutic benefit as compared with other EPO administration methods. Similar compositions and methods can be used for the administration of TPO mimetic of the present disclosure.

Solid dosage forms for oral administration include capsules, tablets, pills, powders, and granules. In such solid dosage forms, the active peptide compound is admixed with at least one pharmaceutically acceptable carrier such as sucrose, lactose, or starch. Such dosage forms can also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration include pharmaceutically acceptable emulsions, solutions, suspensions, syrups, with the elixirs containing inert diluents commonly used in the art, such as water. Besides such inert diluents, compositions can also include adjuvants, such as wetting agents, emulsifying and suspending agents, and sweetening, flavoring, and perfuming agents.

Preparations for parental administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms can also contain adjuvants such as preserving, wetting, emulsifying, and dispersing agents. They may be sterilized by, for example, filtration through bacteria retaining filter, by incorporating sterilizing agents into the compositions, by irradiating the compositions, or by heating the compositions. They can also be manufactured using sterile water, or some other sterile injectable medium immediately before use.

Administration of the TPO mimetic is typically intramuscular, subcutaneous, or intravenous. However other modes of administration such as cutaneous, intradermal or nasal can be envisaged as well. Intramuscular administration of the TPO mimetic can be achieved by using a needle to inject a suspension of the TPO mimetic composition. An alternative is the use of a needleless injection device to administer the composition (using, e.g., Biojector^{™}) or a freeze-dried powder of the TPO mimetic composition.

For intravenous, cutaneous or subcutaneous injection, the TPO mimetic composition can be in the form of a parenterally acceptable aqueous solution which is pyrogen-free and has suitable pH, isotonicity and stability. Those of skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives can be included, as required. A slow-release formulation can also be employed.

Compositions for rectal or vaginal administration are preferably suppositories which may contain, in addition to the active TPO mimetic, excipients such as cocoa butter or a suppository wax. Compositions for nasal or sublingual administration are also prepared with standard excipients well known in the art.

Typically, administration will have a therapeutic and/or prophylactic aim to mitigate the radiation-induced liver disease in a subject prior to, during or following the radiation therapy. In therapeutic applications, the TPO mimetic compositions are administered to a subject during or after the exposure to radiation therapy, and the TPO mimetic compositions are administered in an amount sufficient to cure or at least partially provide mitigation for the radiation-induced liver disease, increase the liver volume or enhance hypertrophy and/or cause engraftment of liver sinusoidal endothelial cells in the liver of the subject. In prophylactic applications, TPO mimetic compositions are administered to a subject susceptible to-or at risk of developing RILDs prior to an exposure to radiation therapy and to enhance liver function post-radiation therapy in a subject in need thereof. In each of these scenarios, the amount of the TPO mimetic compositions will depend on the state and nature of the exposure (e.g., type of radiation therapy, dose and length of exposure), the physical characteristics of the subject (e.g., height, weight, disease state, etc.), and the design of the treatment (e.g., TPOm alone or in combination with another therapeutic agent, etc.)

The pharmaceutically acceptable compositions containing the TPO mimetic are administered to a subject, giving rise to mitigating the radiation-induced liver disease, increasing the liver volume and/or engrafting liver sinusoidal endothelial cells in the liver of the subject. An amount of a composition sufficient to mitigate the disease is defined to be an "effective dose" or an "effective amount" of the composition.

The actual amount administered, and rate and time-course of administration, will depend on the nature and severity of what is being treated. Prescription of treatment, e.g., decisions on dosage etc., is within the responsibility of general practitioners and other medical doctors, or in a veterinary context a veterinarian, and typically takes account of category and dose of the radiation therapy, the condition of the individual patient, the site of delivery, the method of administration and other factors known to practitioners. Examples of the techniques and protocols mentioned above can be found in Remington's Pharmaceutical Sciences, 16th edition, Osol, A. ed., 1980.

In certain embodiments, the TPO mimetic is administered to the subject in combination with liver cells, their progenitors, or stem cells and/or one or more protein factors, such as VEGF-A, VEGF-E, FGF-2, EGF, MMP14, CXCR4 antagonist, SDF1, GM-CSF, GCSF, FLT3, R-Spondin1, and amphiregulin.

In certain embodiments, the TPOm is administered to promote engraftment of liver cells in a subject in need thereof.

As used herein, "engraft" or "engraftment" means to become established as a living part or attachment of a host organ. The graft can be orthotopic or heterotopic to the host organ.

As used herein, "autologous" refers to a biological matter or cells derived from tissues or cells of the subject or host. The ex vivo liver cells to be engrafted into a subject can be autologous.

As used herein, "heterologous" refers to a biological matter or cells derived from the tissues or cells of a different species or different individual of the same species as the subject or host (e.g., allogenic or xenogeneic). The ex vivo liver cells to be engrafted into a subject can be heterologous.

In certain embodiments, the TPOm is administered in combination with hepatic cells, which can be any cells naturally occurring in the liver, including liver sinusoidal endothelial cells (LSECs) and hepatocytes.

In certain embodiments, the TPOm is administered in combination with liver sinusoidal endothelial cells (LSECs). In certain embodiments, the TPOm is administered in combination with hepatocytes. In certain embodiments, the TPOm is administered in combination with LSECs and hepatocytes. LSECs compose a structurally and functionally unique capillary network that vascularizes specific organs, such as liver. The hepatic circulation is predominantly lined by LSECs (Lee, Hepatology 45:817-825 (2007); Klein, Hepatology 47:1018-1031 (2008)), with each hepatocyte residing in close cellular proximity to LSECs.

Cells useful for the invention can be obtained by methods known in the art in view of the present disclosure. For example, hepatocytes can be isolated with a modified collagenase perfusion method from a mammalian organ or tissue, as described by Berry and Friend (Takahashi, M. et al., Gene. Ther., 10:304-313 (2003)). After dissociation, cells can be filtered through a Dacron mesh of a dimension corresponding to the cell of interest and then washed twice at 50×g for 1 min each. Cell viability can be determined by trypan blue dye exclusion. Typically, cells with >90% viability can be used for transplantation, however varying according to site specific protocols or disease state. Ex vivo cells can be adult somatic cells, adult progenitor cells, adult stem cells, embryonic progenitor cells, or embryonic stem cells. Sources of such cells are well known to persons of ordinary skill in the art.

Cells useful for the invention can also be obtained from commercial sources. For example, LSECs, such as SK-HEP-1 cells, can be obtained from recognized depositories, such as the American Type Culture Collection (ATCC, Manassas, Va., USA) as well as other sources. LSECs can also be obtained from pluripotent cells, such as but not limited to human embryonic stem cells, induced pluripotent stem cells and the like by differentiation using methods known in the art.

Suitable growth media for growing cells ex vivo are well known in the art and are disclosed for instance in "Culture of Animal Cells: A Manual of Basic Technique and Specialized Applications" R. I. Freshney, 2010, Wiley-Blackwell. The optimal medium for each type of cells can be obtained from specialized suppliers of the cells (e.g.: ATCC-LGC, MI, Italy; CDC, Atlanta, Ga., USA). For example, the SK-HEP-1 cells obtained from the ATCC and cultured in complete EMEM medium in a humidified cell incubator containing 5% CO₂ at 37° C using gelatin-coated tissue culture flasks. The cells are subcultured by trypsin mediated detachment every 2-3 days following the instructions provided by the ATCC. The density of the LSECs can be from about 5×10⁴ cells/ml to about 5×10⁵cells/ml, from about 2.5 × 10⁴ cells/ml to about 2.5×10⁵ cells/ml, or from about 5×10⁴ cells/ml to about 2×10⁵ cells/ml. In order to obtain optimal treatment, the cell density can, in certain embodiments, be optimized taking into account the nature of the treatment.

In certain embodiments LSEC cells and/or hepatocytes can be harvested from a patient and genetically modified and then transplanted back into the patient to treat a genetic disease that is due to LSEC and/or hepatocyte genetic polymorphisms that result in diseases such as Hemophilia, Alpha1-antitrypsin deficiency, Crigler-Najjar syndrome type I, Familial hypercholesterolemia, Factor VII deficiency, Glycogen storage diseases, Infantile Refsum's disease, Primary oxalosis, and Phenylketonuria.

Ex vivo cells are introduced into the subject in a number which depends upon the cell type and conditions of the organ to be engrafted and the extent of the need for such therapy. For example, ex vivo cells can be injected directly into a target organ of the subject as described previously (Guha, C. et al., Artificial Organs, 25:522-528 (2001)) or administered intraperitoneally or another site of engraftment. They can also be administered intravenously or intraportally (in the case of the liver). For instance, when administering hepatocytes to the rat, under ether anesthesia, the spleen can be exposed, and 5 × 10⁶ hepatocytes suspended in 0.5 ml of RPMI 1640 can be injected into the splenic pulp. Generally, hepatocytes can be administered in suitable medium providing single or divided doses of from 1 × 10⁵to 5×10⁹ cells per treatment. Total dosages, administered singly or as a divided dose, can be from 1 from 1×10⁵to 5×10¹⁰ ex vivo cells/kg of body weight; in some embodiments the total dosages, administered singly or as a divided dose, may be from 1 from 1 × 10⁶ to 1×10⁹ ex vivo cells/kg of body weight; in some embodiments the total dosages, administered singly or as a divided dose, may be from 1 from 1 × 10⁷to 5×10⁸ex vivo cells/kg of body weight.

In some embodiments, the LSECs and/or hepatocytes can be administered by intrahepatic administration using methods known in the art in view of the present disclosure. LSECs induce hepatocytes to proliferate and form new tissue. LSECs themselves can also proliferate to provide vascular support for the regenerating tissue. Preferably, the LSECs are VEGFR2+VE-cadherin⁺VEGFR₃⁺CD34⁻ factor VIII⁺ LSECs. Optionally, the LSECs can be administered together with hepatocytes, and/or a protein factor, such as one or more of CXCR4 antagonist, SDF1, VEGF-A, VEGF-E, FGF-2, EGF, and MMP14.

Other liver cells that can be used in combination with TPOm according to embodiments of the invention include, for example, hepatocytes, hepatic stem cells, pluripotent stem cells, and recombinant liver cells expressing a product of an exogenous polynucleotide sequence. These cells can be obtained and administered to a subject using methods known in the art in view of the present disclosure.

Preferably, a method of the application engrafts ex vivo cells into the liver of a subject in need thereof, and the engrafted cells grow to provide or replace at least 1%, 5%, 10%, 20%, 30%, 40%, 50%, 60%, 70%, 80%, 90%, or 95% (or any range represented therebetween) of the cells of the damaged organ. The cells to be replaced can be of the same cell type as the engrafted cells. More preferably, the engrafted cells grow to a population sufficient in number and activity to ameliorate a condition associated with the damage to the liver or improve a homeostasis (e.g., metabolism of glucose, ammonia, blood lipids, etc.) originally impaired by the damage to the liver. The repopulation by the ex vivo cells typically can provide organ specific parenchymal cells. The ex vivo cell can be autologous or allogeneic to the organ.

Methods known in the art, such as histological analysis, can be used to determine the engraftment. For example, by methods known to one of ordinary skill in the art, engrafted ex vivo cells can be distinguished from endogenous cells by the use of antigenic markers, identifying chromosomal or genomic nucleic acid sequence differences between the engrafted and endogenous cells, fluorescent markers such as GFP, or enzymatic markers such as DPPIV, or according to a functional enzyme present in the ex vivo cell and deficient in the host endogenous cells. Sections of an organ can be embedded in OCT, frozen in liquid nitrogen, and stored at -70° C, or fixed in formalin for paraffin embedding and standard H&E staining. For the liver, Reticulin and trichrome stains can be performed in a standard histopathology laboratory.

A variety of model systems can be used to detect engraftment and repopulation of donor liver cells. For instance, one can use a mouse model, where transgenic beta-galactosidase (B-gal)-expressing (Rosa) C57B1/6 hepatocytes are transplanted into wild-type C57B1/6 mice. Or, DPPIV+ve F344 hepatocytes can be transplanted into congeneric, DPPIV-ve F344 host liver. Since DPPIV is highly expressed in the bile canalicular domain of the hepatocytes, the transplanted cells can easily be detected by enzyme histochemistry. In addition, after characterizing a noninvasive, robust, preparative regimen of hepatocyte repopulation, one can further examine its effectiveness in ameliorating a rodent model of metabolic liver disease, such as the Gunn rat, which is a model for Crigler-Najjar syndrome. Experiments can be simultaneously performed in more than one species.

In the clinical setting, engraftment can be assessed by biopsy with analyses as described above. Additionally, clinical tests can be used to assess the extent to which homeostasis is supported by the engrafted organ. Improvement in one or more clinical parameters related to the functioning of a target or engrafted organ can be used to indirectly assess the efficacy of the engraftment. Suitable clinical tests will vary with respect to the organ.

In certain embodiments, the TPO mimetic is administered to the subject before, after, or simultaneously with the transplantation of LSECs. In certain embodiments, the TPO mimetic is administered to the subject before, after, or simultaneously with the transplantation of hepatocytes. In certain embodiments, the TPO mimetic is administered to the subject before, after, or simultaneously with the transplantation of LSECs and hepatocytes.

The TPO mimetic can also be administered in combination with an administration of one or more protein factors, with or without transplantation of liver cells. Also, as known to those skilled in the art, certain agents can stimulate cell growth or division. Examples of such agents include, but are not limited to, a peptide factor. Preferred stimulus are tissue specific and/or at least stimulate the ex vivo cell type to be engrafted. Stimulus suitable for the invention include protein factors such as: mammalian growth factors (for example HGF, EGF, FGF, VEGF, NGF), Il-6, TNF-alpha, circulating tumor necrosis factor (CTNF), R-spondin 1, Noggin, and TWEAK, growth receptor ligand, and c-met activating antibody. Preferably, the protein factor useful for the invention comprises a factor that stimulate the growth of a LSEC. Examples of such protein factors include, but are not limited to, CXCR4 antagonist, SDF1, VEGF-A, VEGF-E, FGF-2, EGF, MMP14, GM-CSF, GCSF, FLT3, R-spondin1, and amphiregulin. Other preferred protein factors include, for example, thyroid hormone (Parashar, B. et al., Hepatology, 32:206A (2000)), or hepatocyte growth factor (HGF). The growth factors can be wild-type (e.g., human, primate, murine, rat, etc.) or substantially identical to the wild-type factor. It can be produced endogenously by mammalian cells or recombinantly made. The growth factor can be administered locally or systemically. For example, the TPO mimetic can be administered with a CXCR4 antagonist, such as plerixafor.

The TPO mimetic can be administered in combination with a targeted radiation therapy.

In certain embodiments, the TPO mimetic is administered to the subject 24 hours before to 24 hours after, or simultaneously with a targeted radiation therapy. For example, the TPO mimetic is administered to the subject 24, 20, 16, 12, 8, 4, 2, 1, 0.5 or 0.1 hours (or any range represented therebetween) before a targeted radiation therapy, or 24, 20, 16, 12, 8, 4, 2, 1, 0.5 or 0.1 hours (or any range represented therebetween) after a targeted radiation therapy. Preferably, the TPO mimetic is administered to the subject about 24 to about 1 hour (or any range represented therebetween) before the subject is administered with a targeted radiation therapy.

Any suitable dose of the radiation can be used in a method of the application in view of the disclosure in the application and the knowledge in the art. For example, one can perform a dose response study of HIR (e.g., 10, 20, 30 and 50 Gy), in order to identify the lowest dose of HIR that permits effective donor cell repopulation. To investigate the nature of radiation injury to the host hepatocytes, experiments can also be performed with mice that received HIR and TPOm, with or without liver cell transplant and/or protein factor. Animals from various cohorts can be sacrificed at various time points (1d, 2d, 3d, 1 wk, 3 wk, 6 wk and 12 wk) and liver sections can be stained with H&E for histopathological analysis. BrdU and TUNEL staining can be performed for examining hepatocyte proliferation and apoptosis, respectively.

In certain embodiments, the targeted radiation therapy is targeted radiation to the liver, preferably at a dose of 5-70 Gray (Gy), such as 5, 10, 20, 30, 40, 50, 60, or 70 Gy (or any range represented therebetween), in 1 to 10 fractions, such as 1, 2, 3, 4, 5, 6, 7, 8, 9 or 10 fractions. In other embodiments, the targeted radiation therapy is a preparative HIR, preferably a lower dose of HIR, more preferably administered in the clinic using stereotactic radiosurgery (SRS) or 3-D conformal TRT (3-D CRT) techniques. In other embodiments, the targeted radiation therapy is partial liver irradiation administered using modern techniques of IMRT to parts of the liver.

In certain embodiments, the TPO mimetic is administered in combination with a targeted radiation therapy. Preferably, the hypertrophy of the non-irradiated lobe compensates for the hypotrophy of the irradiated lobe of liver. In other embodiments it is administered prior to or after a radiomimetic agent or irradiation therapy.

Any suitable effective amount of TPOm can be used in a method of the application. Such effective amount can be determined using methods known in the art in view of the present disclosure. In certain embodiments, the effective amount of the TPO mimetic is about 1 to about 5 µg/kg, such as 1, 2, 3, 4, or 5 µg/kg (or any range represented therebetween), of body weight of the subject. In preferred embodiments, the effective amount of the TPO mimetic is about 1 to about 3 µg/kg of body weight of the subject.

In certain embodiments, the effective amount of the TPO mimetic is administered to the subject by any one of intravenous, intramuscular, intracutaneous, or subcutaneous injection. In a preferred embodiment, the TPO mimetic is administered by subcutaneous injection.

Following production of the TPO mimetic and optional formulation of the TPO mimetic into compositions, the compositions can be administered to an individual, particularly human or another primate. Administration can be to humans, or another mammal, e.g., mouse, rat, hamster, guinea pig, rabbit, sheep, goat, horse, cow, donkey, monkey, dog or cat. Delivery to a non-human mammal need not be for a therapeutic purpose, but can be for use in an experimental context, for instance in investigation of mechanisms of protecting vascular integrity due to administration of the TPO mimetic.

The TPO mimetic compositions of the invention can be administered alone or in combination with other treatments or additional therapeutic agent, either simultaneously or sequentially dependent upon the condition to be treated.

The term "additional therapeutic agent," as used herein, refers to any compound or therapeutic agent known to or that demonstrates advantageous properties when administered with a TPO mimetic in a method of the application. Examples of such agents can include, but are not limited to, analgesics, antiseptics, other TPO mimetics, other cytokines, soluble mpl receptors, hematopoietic factors, interleukins, protein factors or antibodies, and chemotherapeutic agents. The other cytokines can be stem cell factor (SCF), interleukin 3 (IL-3), CXCR4 antagonist or Flt-3 ligand. See, e.g., Ku et al., Blood, 87:4544-4551 (1996); Sitnicka et al., Blood, 87:4998-5005 (1996). Other agents, such as high doses of corticosteroids, can also be administered in an attempt to decrease intra-hepatic inflammation during an TRT.

### Liver Function Tests

As used herein, the phrase "liver function" refers to a function of the liver, including, but not limited to, protein synthesis such as serum proteins, e.g., albumin, clotting factors, alkaline phosphatase, aminotransferases (e.g., alanine transaminase, aspartate transaminase), 5'-nucleosidase, gamma-glutamyl transpeptidase, etc., synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; excretion function of cholesterol, bile acids, phospholipids and bilirubin; and a hemodynamic function, including splanchnic and portal hemodynamics.

The liver function can be determined using methods known in the art in view of the present disclosure. Standard liver function tests which monitor blood levels of any of alanine aminotransferase; aspartate aminotransferase; alkaline phosphatase; gamma-glutamyl transferase, bilirubin, or ammonia can be used.

Physiological function of repopulated engrafted liver cells, such as hepatocytes and liver sinusoidal endothelial cells (LSECs), can also be tested. The ability of transplanted cells to perform unique hepatocyte biochemical functions, albumin synthesis (albumin immunostaining), glucose metabolism (stain for glucose-6-phosphatase) and gluconeogenesis (glycogen staining) can be examined in fresh frozen sections according to published protocols (Laconi, E. et al., Am. J. Pathol., 153:319-329 (1998)). In Gunn rats, amelioration of hyperbilirubinemia can be used to indicate the degree of repopulation and the physiological function of the transplanted hepatocytes. At various time points (1, 2, 3 and 6 months), animals can be sacrificed, and the function of the engrafted normal hepatocytes can be evaluated by measuring UGT1A1 activity in liver biopsy specimens, performing immunoblot analysis of UGT1A1 and immunohistochemistry to detect donor UGT1A1+ve hepatocytes and by determining the excretion of bilirubin glucuronides in bile. In addition, serum hyaluronic acid in blood, albumin, transferrin, AST, ALT, GGT, GST-pi or alpha-feto protein (AFP) can be measured to examine the normal physiological functions of the liver, and to determine any potential manifestations of radiation injury and tumorigenesis. H & E staining of the liver biopsies can be performed to examine for histopathological evidence of hepatic radiation injury and tumorigenesis.

For example, liver sinusoidal endothelial cells (LSECs) can produce Factor VIII (FVIII), the deficiency of which is characteristic of hemophilia A. Engraftment of normal LSECs that produce FVIII can be confirmed by detection of FVIII in the systemic circulation and liver through a western blot and mRNA profiling of blood and liver derived samples. In the related clinical setting, LSEC engraftment and production of Factor VIII in a hemophilia mouse model (*F8^{tm1Kaz}*) can be assessed with analyses as described above. Additionally, to evaluate the engraftment of LSECs and the restored biological function, a tail vein bleeding test can be performed with regard to the clotting factor, and immunohistochemistry on the livers can also be performed to stain for the presence of FVIII in LSECs.

### EXAMPLES

### Example 1: Effect of TPOm on Non-irradiated Lobe

### Materials and Methods

**Animals:** 8-12-week-old C57BL6 male mice were housed in the Institute for Animal Studies at Albert Einstein College of Medicine and fed regular chow. Animals were weighed in the beginning of the experiment and before tissue collection. All experiments were performed to according to protocols approved by Institutional Animal Care and Use Committee at Albert Einstein College of Medicine.

**Hepatic Irradiation:** Image-guided external beam irradiation was performed using a small animal radiation research platform (SARRP, Xstrahl Inc., Suwanee, GA). Mice were given gastrografin contrast through gavage to improve visualization of the gastrointestinal (GI) tract. After approximately 2 minutes, mice were positioned on a tubular couch and anesthetized with ~2% Isofluorane (Isothesia, USP) in 2 L/min pure oxygen. A cone-beam computed tomography (CBCT) scan was acquired and a treatment plan irradiating the median lobe and right lobe was generated using the MuriPlan software. To minimize toxicity, irradiation was performed using 2 parallel opposed fields. Within the targeted lobes, the position of the isocenter was chosen to minimize irradiation of the GI tract, spine, and heart (the maximum dose to these organs was kept below 10 Gy). Targets were irradiated with 50 Gy X-rays at 220 kVp energy and 13 mA tube current (dose rate of 2.4-2.5 Gy/min) using a 1mm copper filter for beam hardening (Brodin et al., The Radiation Safety Journal, 2015, 109 Supp 3, S190).

**TPOm Formulation Preparation and Dosing:** TPOm was diluted in sterile PBS to a 10 mg/ml stock. The stock was stored at -20°C and allowed to come to room temperature prior to administration. TPOm was administered as a single dose (300 µg/kg) via subcutaneous injection 10 minutes after irradiation.

**Assessments of Targeted Liver Irradiation +/- TPOm:** 50 Gy was administered to the median lobe of the liver of C57BL6 male mice and 10 minutes post-irradiation either saline or 300 µg/kg of TPOm was administered by subcutaneous injection. One month following partial hepatic irradiation, regions of interest were identified in each mouse (n=4 per group).

**Volume of Untreated Caudate Lobe measurements:** With fixed physiologic organ size, non-irradiated liver undergoes hypertrophy in effort to compensate for the irradiated atrophic lobe. The volume of the caudate lobe was measured as a non-irradiated lobe in this experiment.

### Results

### TPOm Causes Hypertrophy of The Non-irradiated Lobe

The mean values and standard deviations for each treatment group are displayed (Figure 1). When comparing non-irradiated caudate lobe size for HIR + saline vs. HIR + TPOm, there was a significant increase in the TPOm treated group (p-value <0.00932). These data demonstrate that TPOm promotes increased liver capacity of the non-irradiated lobe. These effects could be mediated by sinusoidal hepatocyte regeneration or vascular endothelial cell protection/regeneration.

### Example 2: Effect of TPOm +/- Transplanted Liver Sinusoidal Endothelial Cells (LSECs) on Irradiation Induced Liver Injury in Normal and Cirrhotic Livers

### Materials and Methods

**Animals:** 8-12-week-old male and female Di-peptyl peptidase IV (DPPIV) -/knockout mice and 8-12-week-old male and female C57B16 mice were used in this study. Animals were weighed in the beginning of the experiment and before tissue collection. All animals were housed in the Institute for Animal Studies at Albert Einstein College of medicine and fed regular rodent chow. All experiments were performed to according to protocols approved by Institutional Animal Care and Use Committee at Albert Einstein College of Medicine. Animals were made cirrhotic using CC14 administration (intraperitoneal [IP] injections twice a week for at least 11 weeks).

**Hepatic Irradiation:** Image-guided external beam irradiation was performed using a small animal radiation research platform (SARRP, Xstrahl Inc., Suwanee, GA). Mice were given gastrografin contrast through gavage to improve visualization of the gastrointestinal (GI) tract. After approximately 2 minutes, mice were positioned on a tubular couch and anesthetized with ~2% Isofluorane (Isothesia, USP) in 2 L/min pure oxygen. A cone-beam computed tomography (CBCT) scan was acquired and a treatment plan irradiating the median lobe and right lobe was generated using the MuriPlan software. To minimize toxicity, in contrast to Example 1 where two parallel opposed fields were used, irradiation was performed using 2 arcs per liver target with equal dosing weights (likely resulting in more collateral lower dose radiation exposure compared to Example 1). Within the targeted lobes, the position of the isocenter was chosen to minimize irradiation of the GI tract, spine, and heart (the maximum dose to these organs was kept below 10 Gy). Targets were irradiated with 50 Gy X-rays at 220 kVp energy and 13 mA tube current (dose rate of 2.4-2.5 Gy/min) using a 1mm copper filter for beam hardening. (Brodin et al., The Radiation Safety Journal, 2015, 109 Supp 3, S190).

**TPOm Formulation Preparation and Dosing:** TPOm was diluted in sterile PBS to a 10 mg/ml stock. The stock was stored at -20°C and allowed to come to room temperature prior to administration. TPOm was administered as a single dose (300 µg/kg) via subcutaneous injection 10 minutes after the transplant procedure.

### Assessments of Targeted Liver Irradiation +/- TPOm +/- LSEC Transplant

**DPPIV Staining and Imaging:** DPPIV was selected as a marker for engraftment of exogenous LSECs in the DPPIV (-/-) animals. 5 µm frozen liver sections were fixed and stained for DPPIV according to the protocol published by Dabeva et al. (Proc. Natl. Acad. Sci. USA, 1997, 94:7356-7361). Slide imaging was done using a Perkin Elmer P250 High Capacity Slide Scanner.

**Liver Sinusoidal Endothelial Cell (LSEC) Isolation:** Fresh explanted livers were dissociated into single-cell suspensions using a gentle MACS liver dissociation kit (Miltenyi Biotec GmbH #130-105-807). LSECs were isolated from the single-cell suspension using CD146 microbeads (Miltenyi Biotec GmbH #130-092-007).

**Cell Transplantation:** LSEC transplantation is a novel technique that requires a strong growth stimulus for effective liver repopulation (Guha et al., Hepatology, 2002, 36:354-62; Laconi et al., Am. J. Pathol., 1998, 153:319-29). Following isolation of LSECs, 500 × 10⁵ LSECs are transplanted via intrasplenic injection into healthy adult mice anesthetized with Isoflurane (Isothesia, USP) 24 hrs post HIR. Buprenorphine is given post-op as an analgesic.

**Single-Photon Emission Computed Tomography (SPECT) Scan:** SPECT with a radiotracer to assess the progression of RILD in-vivo has been developed and utilized in this work. Animals were brought to the laboratory the night before and allowed to acclimate to the ambient noise of the equipment. The animals were allowed to rest for at least 10 minutes under isoflurane anesthesia. The dose of ^{99m}Tc sulphur colloid was drawn from a 10 mCi dose held in a 2-mL syringe behind the lead. Activity was drawn up to 0.500 - 0.600 mCi up to 0.2 mL volume max and injected via the retro-orbital sinus. The needle was applied with positive pressure at a rate of approximately 3 seconds per 0.1 mL volume. The animal was then immediately placed in the tunnel of the SPECT/CT scanner. A CT scout view was taken with very minimal radiation exposure to the animal. This was to align the animal in the field of view (FOV). The CT was set on medium resolution and was measured by a dosimeter and read by a third-party company, at an exposure of 5 mRem/hr for each CT scan/animal. The animal was then moved by the gantry into the SPECT FOV and then initiated. The counts were then collected in SPECT mode. This was for an additional 20 minutes as both SPECT detector heads, 180 degrees apart rotate around the animal while in the tunnel. Animals were observed at the end of the study for recovery and then placed into a special room for radiation decay. Technetium 99m (⁹⁹mTc) has a 6-hour half-life which implies a 60-hour hold before releasing the animal. CT Acquisition was performed via total rotation 360 degrees, 180 rotation steps, exposure time 200ms/1 frame voltage set to 80 kV current was set to 0.5 mA, Transaxial FOV: 59.45mm, Axial FOV: 68.26mm System Magnification set to medium resolution, effective pixel size 69 microns. CT Reconstruction was performed using a standard Houndsfield reconstruction protocol, accept all defaults.

**SPECT Acquisition:** Collimator: 5-hole mouse whole-body (5-MWB-1.0) at 30mm radius of rotation Transaxial FOV 38mm, Maximum resolution 1.3mm, Acquisition mode set to: SPECT Scan Photo Peak set to: 140keV, Isotope: Technetium 99m (99mTc), number of revolutions: 0.5Angle between projection: 3.7, Total projections: 40, 1st step acquisition time: 20seconds, Estimated scan time: 19 minutes. SPECT Histogram: Acquisition mode: SECT Scan, Lower level discriminator, set 126keV, Upper discriminator set to 154keV, Data format: Intel/VAX 4byte integer SPECT Reconstruction: Detectors that are enabled: 1 and 2, Reconstruction type: Ordered subset expected maximization three dimensional (OSEM3D), Iterations: 8, Subsets: 4.

**Image and Data Analysis:** To further investigate the character of tissue exposed to radiation injury and the extent of vascular damage and recovery 2 months after radiation treatment, the volume of tissue showing reduced perfusion using an in-house developed semiautomatic quantification software, implemented using MATLAB (The MathWorks Inc., Natick, MA) was measured. The software used a simple thresholding algorithm designating the volume of reduced perfusion as that with 15-80% of tracer uptake compared to the non-irradiated lobe. The software performance was validated using SPECT/CT scans from animals with a known volume of irradiated liver tissue or receiving no liver irradiation.

**Statistical Analysis:** Statistical analysis was performed using PRISM 7 (GraphPad) statistical analysis software. Data are presented as mean ± standard deviation. Ordinary one-way analysis of variance was used for comparisons. All p values are reported.

### Results

**TPOm Cause Proliferation And Engraftment of Transplanted LSECs In Irradiated Liver**

To test the efficacy of TPOm as an agent that can cause LSEC engraftment, DPPIV-/- knockout mice received 50GY hepatic irradiation (HIR) to the median and right lobes of the liver using external-beam CT-guided x-ray irradiation, and LSECs that express DPPIV (Figure 2A). DPPIV staining in a corkscrew pattern indicates repopulation of LSECs between hepatocytes in the hepatic sinusoids (Figure 2B and Figures 3A - 3F).

LSECs do not repopulate the irradiated liver without a growth stimulus (Figure 3C) but when given a strong growth stimulus, transplanted cells repopulate the liver sinusoids (Figure 3D). When TPOm is used as a growth stimulus, massive repopulation of irradiated tissue is observed (Figures 3E and 3F). In 6/7 animals receiving LSEC + TPOm + HIR some level of hepatic repopulation is seen (Figure 3F).

### TPOm-Based Treatments Reduce Liver Injury in Irradiated Tissue Following Hepatic Irradiation

Radiation generates free radicals and damages genomic DNA, which leads to apoptosis and cell senescence. Irradiated liver tissue shrinks and the hepatostat compensates by increasing the volume of healthy liver tissue. To investigate how TPOm-based treatments affect radiation-induced liver injury, individual liver lobes were carefully dissected and weighed. Figures 4A and 4B show the relative changes in liver lobe size normalized to the total size of the liver and the weight of the animal. There was a trend for an increase in the volume of the non-irradiated left lobe at 2-months post-HIR in animals receiving HIR and then LSECs +/- RWJ-800088 at 24-hours post-HIR (Figure B). In addition, there was a trend for a reduction in the size of the irradiated lobes (median and right) in the group treated with RWJ-800088 and HIR only (Figure 4A). The trend for a reduction in the size of the irradiated lobe with RWJ-800088 + HIR alone may be a consequence of increased cell death due to increased proliferation of DNA irradiation-damaged cells.

Liver vascular injury and corresponding sinusoidal obstruction in in the liver is reduced in animals treated with RWJ-800088 alone and in combination with LSEC transplantation. SPECT can be used to visualize and quantify perfusion changes in the liver by measuring the signal of sulphur-colloid linked Tc⁹⁹ (SC-Tc99), which is selectively taken up by the liver Kupffer cells within 15 minutes of injection into circulation. Animals receiving 50GY HIR to the median lobe have a mean residual defect volume of 71.4% in the irradiated region 2 months post HIR (Figure 5B and 5E). In animals with normal livers treated with RWJ-800088 either 2 or 24 hours prior to irradiation, the mean residual defect volume is similar to the non-irradiated animals (Figure 5 E). In animals with normal livers that received RWJ-800088 and LSEC transplantation 24 hours after irradiation, the residual defect volume in the irradiated area at 2 months post HIR was 18.2%, (p =0.03) (Figure 5C and 5E). A similar protective effect was observed in animals with cirrhotic livers that received RWJ-800088 and LSEC transplantation 24-hours post irradiation (Figure 5D).

### Example 3: Effect of TPOm or Romiplostim +/- Transplanted Liver Sinusoidal Endothelial Cells (LSECs) Following Irradiation

### Materials and Methods

The methods were identical to those described above with the exception that the LSEC transplant was administered 4-5 days post irradiation and the TPOm or Romiplostim were administered approximately 10 minutes post LSEC transplant.

### Results

### TPOm and Romiplostim Cause Proliferation and Engraftment of Transplanted LSECs In Irradiated Liver

Following administration of TPOm or romiplostim LSEC engraftment was observed in DPPIV-/- knockout mice that received 50GY hepatic irradiation (HIR) and an LSEC transplant (Table 1). DPPIV staining in a corkscrew pattern indicates repopulation of LSECs between hepatocytes in the hepatic sinusoids (Figure 6).

**Table 1: Summary of LSEC engraftment following TPOm or Romiplostim administration following targeted liver irradiation (59 Gy)**

| **Results** | **TPOm** | **Romiplostim** |
|---|---|---|
| Engraftment when LSEC transplant is administered 24 hours post irradiation | Yes | Yes |
| Engraftment when LSEC transplant is administered 4-5 days post irradiation | Yes | Yes |

### Example 4: Development of Mouse Cirrhosis Model to Evaluate the Effect of TPOm on Liver Regeneration in Cirrhotic Mice

**Objective:** To develop a mouse cirrhosis model to enable an evaluation of the effect of RWJ-800088 on the liver regeneration in cirrhotic mice after a single hepatic irradiation (HIR) at 10 Gy. Multiple dose levels of CCl₄ will be evaluated to determine an appropriate dose level to maintain liver injury without causing mortality. The conditions used in this study are used in Experiment 5.

### Materials and Methods

**Animals:** Male C57BL/6 DPPIV knockout male mice (10-14 weeks) were injected intraperitoneally with carbon tetrachloride (CCl₄) twice a week for a minimum of 12 weeks until a maximum of when 5 out of 30 mice perish from the CCl₄ injection. Immediately after, the animals were randomly distributed into the experimental groups.

**Hepatic Irradiation (HIR):** Image-guided external beam irradiation was performed using a small animal radiation research platform (SARRP, Xstrahl Inc., Suwanee, GA). Mice were given gastrografin contrast through gavage to improve visualization of the gastrointestinal (GI) tract. After approximately 2 minutes, mice were positioned on a tubular couch and anesthetized with ~2% isofluorane (Isothesia, USP) in 2 L/min pure oxygen. A cone-beam computed tomography (CBCT) scan was acquired and a treatment plan irradiating the median lobe and right lobe was generated using the MuriPlan software. To minimize toxicity, irradiation was performed using 2 arcs per liver target with equal dosing weights. Within the targeted lobes, the position of the isocenter was chosen to minimize irradiation of the GI tract, spine, and heart (the maximum dose to these organs was kept below 10 Gy). Arc positions coordinates are described below **(Table 2**). Targets were irradiated with 50 Gy X-rays at 220 kVp energy and 13 mA tube current (dose rate of 2.4-2.5 Gy/min) using a 1mm copper filter for beam hardening.

**Table 2: Parameters of Hepatic Irradiation**

| | **Couch Start** | **Couch End** | **Gantry start** | **Gantry end** |
|---|---|---|---|---|
| Median Lobe Arc 1 | 20 | 20 | -110 | 100 |
| Median Lobe Arc 2 | -20 | -20 | -110 | 100 |
| Right Lobe Arc 1 | 40 | 40 | -20 | 120 |
| Right Lobe Arc 2 | -40 | -40 | -20 | 120 |

**Preparation of the Test Articles:** Stock solution of RWJ-800088 was prepared by weighing out 1 mg of RWJ-800088 and dissolving it in 1 mL of sterile normal saline (Control Article). The stock solutions were stored at -80°C. On the day of administration, the stock solution was thawed to room temperature and a dosing solution was prepared from the stock solution at the appropriate concentrations for dosing. The dosing solution was vortexed to ensure homogeneity of the dosing solution.

**Methods:** Mice were treated with CCl₄ (two injections at 40% v/v and the remaining injections at 10% v/v for 11 weeks were evaluated to determine an appropriate dose level to maintain liver injury without causing mortality.

**Time Points for the Experiment:** Mice were treated with CCl₄ to induce cirrhosis. The liver of the cirrhotic mouse was irradiated by using an X-ray irradiator (SARRP) at 50 Gy (set as time 0), followed by administration of RWJ-800088. After 3 months, the mice were sacrificed to collect blood and liver tissues for further analysis.

**Timing/Dose of Drug Administration:** RWJ-800088 at 300 µg/kg was administrated subcutaneously at 10 min after HIR as shown in **Table 3** below:

**Table 3: Timing and Dose of Drug Administration**

| **Group** | **Test Article** | **Timing** | **Liver RT** | **Gender** | **No of Mice** |
|---|---|---|---|---|---|
| 1. | Sham | N/A | 0 Gy | M | 3 |
| 2. | Vehicle | +10 min after HIR | 50 Gy RL + pML | M | 3 |
| 3. | RWJ-800088, 300 µg/kg | RWJ-800088 +10 min | 50 Gy RL + pML | M | 3 |
| 4. | RWJ-800088, 300 µg/kg | RWJ-800088at +10 min, LSEC transplantation at +1 day after HIR | 50 Gy RL + pML | M | 3 |

### Endpoints evaluated:

- E1 - Liver weight assessed at time of sacrifice (3 months post-HIR)
- E2 - Liver histology at time of sacrifice (3 months post-HIR)

**Clinical Observations:** The animals were monitored daily by both the veterinary and research staff to ensure that animals meeting criteria for euthanasia were removed from the study.

**Body Weights:** Animals were weighed prior to the start of the experiment, on a weekly basis during the experimental period, and on the day of euthanasia .

### Results

Mice were first injected with 40% CCl₄ (v/v) in olive oil, however many of the mice died after 2 subsequent injections. The dose was then reduced to 10% for the remainder of this experiment.

### Example 5: Effect of TPOm +/- Transplanted Liver Sinusoidal Endothelial Cells (LSECs) and TPOm +/- Plerixafor on Irradiation Induced Liver Disease in Cirrhotic Mice

**Objective:** To evaluate the effect of RWJ-80088 + Liver Sinusoidal Endothelial Cells (LSEC) & RWJ-80088 + Plerixafor (a selective inhibitor of CXCR4 administered pre- and post-irradiation) on radiation induced liver disease as measured by SPECT-CT in cirrhotic mice after single hepatic irradiation (HIR) at 50 Gy. AdHGF is included as a positive control.

### Materials and Methods

The methods were identical to Experiment 4 as described above with the exception that the CCl₄ dose was increased to 20% in olive oil over 12 weeks. To evaluate the effect of RWJ-800088 + Liver Sinusoidal Endothelial Cells (LSEC), RWJ-800088 + Plerixafor (a selective inhibitor of CXCR4 administered pre- and post-irradiation) on radiation induced liver disease as measured by SPECT-CT in cirrhotic mice after single hepatic irradiation (HIR) at 50 Gy. AdHGF was a positive control. These data aim to provide preclinical support for conduct of clinical studies in patients with underlying liver disease.

**Preparation of the Test Articles:** The dosing solution of RWJ-800088 was prepared as described in Experiment 4. In addition, adenoviral vectors were harvested by cell lysis and purified by CsCl gradient centrifugation. The number of viral particles and infectious units' ratio was determined by infecting 293 cells at various dilutions, followed by immunocytochemical staining for adenoviral hexons. The infectious titer was calculated from the number of plaques formed per 1,000 viral particles. One day after cell transplantation, 1 ×1011 infectious units of adenovirus human hepatic growth factor (AdHGF) was administered by tail vein injection.

Plerixafor (Mozobil) from Genzyme Corporation was packaged in a vial with concentration of 20 mg/ml. It is a bicyclam compound as CXCR4 chemokine receptor antagonist and has been shown in multiple earlier studies to rapidly and effectively increase the number of stem cells in circulation.

**Time Points for the Experiment:** Mice were first treated with CCl₄ for 11-weeks to induce cirrhosis. The liver of cirrhotic mouse was irradiated by using an X-ray irradiator (SARRP) at 50 Gy (set as time 0), followed by administration of RWJ-800088 in the combination of Plerixafor, liver sinusoidal endothelial cell (LSEC) transplantation, or adenovirus hepatocyte growth factor (AdHGF). After 4 months, mice were sacrificed to collect blood and liver tissues for further analysis.

**Timing/Dose of Drug Administration:** RWJ-800088 at 300 µg/kg was administered subcutaneously at 10 min after HIR. Plerixafor at 5 mg/kg was administered subcutaneously at various time points as indicated the following Table 4. LSECs were transplanted through intrasplenic injection at 48 hours after HIR. AdHGF was administered intravenously at 72 hours after LSEC transplantation.

**Table 4: Timing and Dose of Drug Administration**

| **Group** | **Treatment** | **Radiation** | **Gender** | **n** |
|---|---|---|---|---|
| 1 | Vehicle | 50 Gy RL + pML | M | 4 |
| 2 | RWJ-800088 (300 µg/kg + 10 min after HIR); Plerixafor (5 mg/kg + 10 min and + 48 hours after HIR) | 50 Gy RL + pML | M | 3 |
| 3 | RWJ-800088 (300 µg/kg + 10 min after HIR); Plerixafor (5 mg/kg - 7 days before and 10 min after HIR) | 50 Gy RL + pML | M | 3 |
| 4 | RWJ-800088 (300 µg/kg + 10 min after LSEC transplant) | 50 Gy RL + pML | M | 4 |
| | LSEC (5 × 10⁵/mouse + 48 hours after HIR) | | | |
| 5 | LSEC (5 × 10⁵/mouse + 48 hours after HIR) | 50 Gy RL + pML | M | 4 |
| | AdHGF (1 × 10¹¹ viral particles/mouse +72 hours after LSEC transplant) | | | |

### Endpoints evaluated:

- E1 - SPECT-CT of liver
- E2 - Liver weight assessed at time of sacrifice (4 months post-HIR)
- E3 - Liver histology at time of sacrifice (4 months post-HIR)

### Results

Plerixafor administration post injury together with RWJ-800088 increased the hypertrophy of the non-irradiated lobe as evidenced by the ratio of the weight of the non-irradiated lobe to irradiated lobe. Plerixafor administered pre- and post-irradiation reduced the extent of protection against RILD and the degree of hypertrophy. RWJ-800088 showed a trend toward protection against RILD similar to AdHGF (positive control). See Table 5 and Figure 7.

**Table 5: Ratio of the weight of the non-irradiated right lobe to the weight of the irradiated left lobe**

| **Group** | **Average Ratio of Right Lobe to Left Lobe Weight** | **SD** | **n** |
|---|---|---|---|
| HIR | 1.14 | 0.33 | 3 |
| HIR + TPOm + Plerixafor Post | 2.17 | 0.63 | 3 |
| Plerixafor Pre + HIR + TPOm + Plerixafor | 0.93 | 0.18 | 2 |
| Post | | | |
| HIR + LSEC + TPOm | 1.35 | | 1 |
| HIR + LSEC + AdHGF | 1.79 | 0.94 | 4 |

Quantifying the extent of RILD by SPECT-CT (Figure 8), mice treated with RWJ-800088 with LSEC transplants or plerixafor were protected against RILD and showed an increase in hypertrophy of the non-irradiated lobe. Plerixafor administration both pre- and post-irradiation reduced the extent of protection against RILD and the degree of hypertrophy.

### Example 6: Effect of TPOm +/- Transplanted Liver Sinusoidal Endothelial Cells (LSECs) on Production of Factor VIII Following Irradiation Therapy to The Liver of Hemophilia A Mice

**Objective:** To evaluate the effects of RWJ-800088 on LSEC engraftment and production of Factor VIII in a Hemophilia A mouse model (F8tm1Kaz ) following targeted irradiation of 50 Gy to the superior right and median lobe of the liver. AdHGF, which cannot be used in humans for safety reasons, was used as a positive control because it has been shown to cause LSEC engraftment following targeted liver irradiation and LSEC transplant.

### Materials and Methods

**Animals:** 8-16-week-old male C57BL6 mice, 8-16-week-old female and male B6129SF2/J mice (donor for isolating LSECs) and 8-16-week-old female and male Hemophilia mice (*F8^{tm1Kaz}*) mice were used in this study. Mice were housed in groups of up to 5 in micro-isolator cages on stainless steel racks (Allentown, Inc., Allentown, NJ) with a total floor space of 500 cm². The cages utilized an external water bottle and a dual feeder rack (Fisher Scientific, Hampton, NH). These cages were stored in the storage rack which provided temperature, humidity, and ventilation control to each individual cage (Allentown, Inc.). Bed-o'Cobs 1/8" (Fisher Scientific) were used as bedding material in each cage.

**Methods:** The methods were identical to Experiment 5 as described above; including the preparation of test articles (solutions of RWJ-800088 and AdHGF), isolation and transplant of LSECs, and Hepatic irradiation.

**Time Points for the Experiment:** The livers of mice were irradiated at 50 Gy by using an X-ray irradiator (SARRP). At 24 hours post-irradiation, the LSECs (1 × 10⁶ cells /mouse) were injected to the mouse through tail vein. An incision was made in the tail from each mouse at day 60 after hepatic irradiation (HIR) and subjected to the bleeding test.

**Timing/Dose of Drug Administration:** RWJ-800088 (300 µg/kg) was administrated subcutaneously at 10 min after LSEC transplantation. AdHGF at 1 × 10⁹ PFU/mouse was administrated intraperitoneally at 24 hours after LSEC transplantation. The treatment groups were indicated in the following Table 6.

**Table 6.**

| **#** | **Mouse Strain** | **Hepatic Irradiatio n** | **N Males** | **Test Article** | **LSEC (cells/µ L)** | **Dose Volume LSEC (µL)** | **Test Article (mg/mL; PFU)** | **Test Article (µg/kg; PFU)** |
|---|---|---|---|---|---|---|---|---|
| B | C57BL6 | None | 5 | | - | - | - | 0 |
| D | B6129SF2 (LSEC Donor)* | None | 15 | | - | - | - | 0 |
| H | Hemophilia A | None | 15 | | - | - | - | 0 |
| G | Hemophilia A | HIR 50 Gy | 15 | | - | - | - | 0 |
| A | Hemophilia A | HIR 50 Gy | 15 | RWJ-800088 | 1 × 10⁶ | 200 µl | 1 mg/ml | 300 |
| T | Hemophilia A | HIR 50 Gy | 15 | AdHGF | 1 × 10⁶ | 200 µl | 1011 PFU | 109 |

| | | | | | | | | |
|---|---|---|---|---|---|---|---|---|
| * 10 were used for LSEC isolation and 5 were used for bleeding tests and other endpoint analyses | | | | | | | | |

**Endpoints of Study:** A tail bleeding test was performed in which the extent of bleeding and time to clotting over the course of 30 minutes was evaluated. FVIII protein levels were evaluated by Western blot and mRNA profiling in the blood and liver to evaluate the extent to which there was engraftment of functioning LSECs that produce FVIII. Immunohistochemistry on the livers was also performed to stain for the presence of FVIII in LSECs.

### Results

### Drabkin's bleeding time assay in Hemophilia A mice

The tail bleeding assay was performed by cutting the tail and then immersing the tail in 2.0 ml of Drabkins reagent. Bleeding stopped before 10 min in the control groups and in the HIR + LSECs + AWJ-800088 or AdHGF groups, whereas the nontreated and HIR treated Hemophilia A groups did not stop bleeding. The comparison of the haemoglobin concentration in the collection tube when using HIR + LSECs + RWJ-800088 and HIR + LSECs + AdHGF showed a significant decrease in haemoglobin loss compare to FVIII deficient model.

### Survival after trail bleeding

Ninety days post treatment, mice were subjected to tail bleeding challenge. As shown in Figure 9, after tail bleed challenge, 100% of the control Hemophilia A mice died within 5 days while the HIR + LSECs + RWJ-80088 treated group showed 100% survival compared to control 30 days post tail bleed challenge. Note that these survival curves are only for animals that were subjected to the Drabkin's bleeding test. The AdHGF mice died day 5, day 15 and day 23 compared to control groups (cause of death not known).

In addition, ELISA analysis shows prolonged expression of factor VIII (FVIII) in the treated HIR+ LSECs + JNJ-26366821 or AdHGF groups compared to non-treated Haemophilia A mice in pg/mL for plasma (Figure 10A) or pg/g for liver (Figure 10B).

Similar results were also obtained from the immunofluorescence imaging, which evaluated liver slides stained for Factor VIII, LYVE-1 - selective antibody for liver sinusoidal endothelial cells, DAPI -DNA stain showing cells, and merged areas for FVII + LYVE-1 (Figure 11).

LSEC transplantation together with administration of RWJ-800088 following targeted hepatic irradiation (HIR) promoted engraftment of functional LSECs and FVIII production in Hemophilia A mice. This was demonstrated with a functional bleeding assay in which there was reduced bleeding and clotting time similar to non-Hemophilia A controls. After the tail bleed challenge, 100% of the non-treated and HIR treated Hemophilia mice died within 5 days while the HIR + LSECs + RWJ-800088 treated hemophilia mice showed 100% survival out to 30 days. There was also increased levels of Factor VIII mRNA and activated protein in plasma and liver of the HIR + LSECs + RWJ-800088 treated hemophilia mice. The presence of Factor VIII in the liver was also confirmed by immunohistochemistry and immunofluorescent imaging. This study provides proof of concept for the potential to use a thrombopoietin mimetic such as RWJ-800088 in combination with an LSEC transplant and HIR to cure Hemophilia A.

Administration of RWJ-800088 in combination with LSECs that produce FVIII following targeted hepatic irradiation (HIR) to the superior right and median lobe of the liver of Hemophilia A mice promoted engraftment of functioning LSECs that produce FVIII to a sufficient extent that they enable clotting and survival following a tail vein bleeding test. The successful engraftment of functioning LSECs was also confirmed by measurement of FVIII protein levels and mRNA in circulating blood and liver as determined by ELISA, western blot and immunohistochemistry. This study provides proof of concept for the potential to use a thrombopoietin mimetic in combination with an LSEC transplant and HIR to cure Hemophilia A.

### Example 7: Effect of TPOm and Romiplostim on Engraftment of Transplanted Liver Sinusoidal Endothelial Cells (LSECs) and Hepatocytes Following Irradiation to DPPIV knockout Mice

**Objective:** To evaluate the effects of RWJ-800088 and Romiplostim on the engraftment of the transplanted liver sinusoidal endothelial cells (LSECs) and hepatocytes following hepatic irradiation (HIR).

### Materials and Methods

**Animals:** 8-12-week-old male Di-peptyl peptidase IV knockout (*Dppiv*^{*-*/*-*}) mice and 8-12-week-old male C57BL/6 mice were used in this study.

**Methods:** The methods were identical to Experiment 5 as described above; including the preparation of test articles (solutions of RWJ-800088), isolation and transplant of LSECs, and Hepatic irradiation. In addition, Romiplostim was purchased from pharmacy and came in 250 µg powder. It was reconstituted per manufacturers description using solvent for solution for injection. After reconstitution, a deliverable volume of 0.5 mL solution contained 250 µg of romiplostim (500 µg/mL).

### Experimental Design

**Study A:** Animals were weighed in the beginning of the experiment. Male C57BL/6 and DPPIV KO mice (8-12-weeks old) received 50 Gy to the superior right and median lobes of the liver using the Small Animal Irradiator Platform (SARRP system).

The mice were transplanted with DPPIV⁺ 5×10^5 liver sinusoidal endothelial cells (LSECs) intrasplenically 4 or 5 days later. The mice were then injected with 300 µg/kg of RWJ-800088 or Romiplostim intraperitoneally 10 minutes after LSEC transplantation as shown in Table 7.

**Table 7**

| **Group #** | **Treatment** | **Targeted Radiation (Gy)** | **Dose (µg/kg)** | **Dose Volume (µL)** | **LSECs (cells/mouse)** | **N** |
|---|---|---|---|---|---|---|
| 1 | RWJ-800088 | 50 | 300 | 100 µl | 5×10^5 | 4 |
| 2 | Romiplostim | 50 | 300 | 100 µl | 5×10^5 | 4 |

The mice were then monitored until they made a full recovery and returned to the cage. The mice were followed for 2 and 5 months and necropsied. The liver was excised and prepared for the frozen section. The immunostaining of DPPIV activity was performed to examine the repopulation of the transplanted DPPIV⁺ cells in the liver.

**Study B** (Romiplostim only Study): To study the engraftment and repopulation efficiency of hepatocyte (HT) alone and HT/LSECs with the presence of romiplostim in the preparative hepatic radiated DPPIV KO mouse model. The study design is shown in Table 8:

**Table 8**

| Group | Group | Gender | Number of Animals | Transplants | ROMI Dose (µg/kg) 3 | H₂O Dose (mg/kg) 3 |
|---|---|---|---|---|---|---|
| 1 | HIR + HT + Vehicle | M/F | n = 2 | Hepatocyte 1X10 ⁶ | 0 | 100 ul for 3 days |
| 2 | HIR + HT + ROMI | M/F | n = 2 | Hepatocyte 1X10 ⁶ | 100 ug/kg for 3 days | 0 |
| 3 | HIR + HT + LSEC + ROMI | M | n = 1 | Hepatocyte 1X10 ⁶ + LSECs SX10 ⁵ | 100 ug/kg for 3 days | 0 |
| 4 | NO HIR + HT + Vehicle | M/F | n = 2 | Hepatocyte 1X10 ⁶ | 0 | 100 ul for 3 days |
| 5 | NO HIR + HT + ROMI | M/F | n = 2 | Hepatocyte 1X106 | 100 ug/kg for 3 days | 0 |
| 6 | NO HIR + HT + LSEC + ROMI | M | n = 1 | Hepatocyte 1X10 ⁶ + LSECs SX10 ⁵ | 100 ug/kg for 3 days | 0 |

Groups 1-3: Radiation will be administered on Day 1. Hepatocyte (HT) alone and Hepatocyte (HT) + LSEC transplantation will occur 24 hours after irradiation. Post transplantation romiplostim will be administered for 3 days for the above respective groups.

Groups 4-6: Hepatocyte (HT) alone and Hepatocyte (HT) + LSEC transplantation performed in non-radiated mice. Following transplantation romiplostim will be administered for 3 days for the above respective groups.

### Results

**Study A:** The immunostaining of DPPIV activity was performed in the collected liver tissues of the surviving animals. At 2 months after HIR, when RWJ-800088 was administered in conjunction with HIR and LSECs, repopulation of LSECs in the irradiated liver tissue was observed and similarly with romiplostim treatment, compared to no positive staining in the liver of the normal DPPIV KO mouse. In addition, there was a difference in staining pattern between RWJ-800088- and romiplostim-treated mouse. It is related to architecture of frozen tissues where some may not be as well maintained compared to others.

We also stained 4 different liver lobes of another mouse treated with RWJ-80088 at 2 months after HIR. All 4 lobes showed positive staining, while the median lobe had the least area covered with the DPPIV-positive cells. At 5 months after HIR, only two RWJ-80088-trreated mice were survived for the liver staining. The median, right, and left lobes of one mouse showed extensively positive staining with DPPIV, but not much in the caudate lobe. In the other mouse, the median and right lobes showed the strong DPPIV staining, but not much in the left and caudate lobes. These results indicated that transplanted LSECs to the HIR mice treated with RWJ-800088 are well repopulated and stably resided in the liver.

In this study, the radiation is targeted to the median and right lobes of the liver, which shows the intensive DPPIV staining. In contrast, there is not much transplanted LSECs in the caudate lobe. This result indicates that radiation precondition is important factor to recruit transplanted LSECs.

**Overall Summary Results across Study A and Study B:** The following Table 9 summarizes the population of hepatocytes and LSECs in the different treatment groups across Study A and Study B.

**Table 9**

| | **Hepatocytes** | **LSECs** |
|---|---|---|
| Vehicle | None | None |
| Vehicle + HIR | Minimal to Low | Minimal to Low |
| RWJ-800088 + HIR | Substantial repopulation at 16 weeks with 1 dose at 10 min post-transplant 24 hours post HIR | Substantial repopulation at 16 weeks with 1 dose at 10 min post-transplant 24 hours post HIR |
| Romiplostim + HIR | Substantial repopulation at 16 weeks with 3 doses at 10 min following transplant | Substantial repopulation at 16 weeks with 1 dose at 10 min following transplant |

These data demonstrate that both TPOm and romiplostim can separately act to promote the engraftment of LSECs, hepatocytes, or LSECs and hepatocytes in mice following hepatic irradiation as evaluated by histology. These results suggest that TPOm and romiplostim have a regenerative effect on the liver vasculature following HIR.

## Claims

1. A thrombopoietin (TPO) mimetic comprising the amino acid sequence of SEQ ID NO: 1 for use in a method of mitigating a targeted radiation therapy-induced liver disease or a radiomimetic agent-induced liver disease in a subject in need thereof.

2. The TPO mimetic for use of claim 1, wherein the TPO mimetic is administered in combination with the targeted radiation therapy or the radiomimetic agent.

3. The TPO mimetic for use of claim 1 or 2, wherein the targeted radiation therapy-induced liver disease is selected from the group consisting of hepatomegaly, hepatic necrosis, elevated liver enzymes, hepatic sinusoidal obstruction syndrome (SOS), hepatic central venous occlusive disease (VOD), and hepatic fibrosis.

4. The TPO mimetic for use of any one of claims 1-3, wherein the method (i) promotes liver hypertrophy of non-irradiated liver, (ii) promotes engrafting of liver sinusoidal endothelial cells (LSECs) and/or hepatocytes in irradiated liver, and/or (iii) reduces liver damage either alone or in combination with administration of LSECs.

5. The TPO mimetic for use of any one of claims 1-4, wherein the TPO mimetic is administered to the subject in combination with at least one of liver cells and a protein factor, wherein the liver cells comprise at least one selected from the group consisting of liver sinusoidal endothelial cells (LSECs), hepatocytes, hepatic progenitor cells, hepatic stem cells, pluripotent stem cells, and recombinant liver cells expressing a product of an exogenous polynucleotide sequence; preferably the liver cells comprise LSECs, and optionally hepatocytes, and wherein the protein factor comprises a growth factor and optionally a hepatocyte growth factor, more preferably, the growth factor comprises one or more of a CXCR4 antagonist, SDF1, VEGF-A, VEGF-E, FGF-2, EGF, GM-CSF, GCSF, FLT3, Rspondin-1, amphiregulin, and MMP14.

6. The TPO mimetic for use of claim 5, wherein the TPO mimetic is administered to the subject in combination with LSECs, which are transplanted to the subject, before, after or simultaneously with the administration of the TPO mimetic.

7. The TPO mimetic for use of claim 4, wherein the TPO mimetic is administered to the subject in combination with LSECs and/or hepatocytes, which are administered to the subject together with a protein factor, and optionally hepatocyte growth factor; and further, more preferably, the protein factor is a stem cell growth and/or differentiation factor comprising one or more of CXCR4 antagonist, SDF1, VEGF-A, VEGF-E, FGF-2, EGF, GM-CSF, GCSF, Rspondin-1, FLT3, amphiregulin, and MMP14.

8. The TPO mimetic for use of claim 7, wherein the TPO mimetic is administered to the subject, before, after or simultaneously with a transplantation of the LSECs and/or hepatocytes.

9. The TPO mimetic for use of any one of claims 1-8, wherein the subject is treated with a stereotactic radiation therapy or transarterial chemoembolization (TACE) for the liver disease.

10. The TPO mimetic for use of claim 9, wherein the subject is treated with targeted radiation at a dose of 10-70 Gray (Gy) in 1 to 10 fractions.

11. The TPO mimetic for use of any one of claims 1-10, wherein the subject is treated for a liver tumor or a liver metastasis, preferably a liver cancer, more preferably hepatocellular carcinoma (HCC).

12. The TPO mimetic for use of any one of claims 1-10, wherein the subject is treated with a preparative hepatic irradiation (HIR) for engraftment of liver cells.

13. The TPO mimetic for use of any one of claims 1-10, wherein the subject is treated with a radiation therapy for a gastrointestinal cancer or the subject is treated with a preparative irradiation for bone marrow transplant.

14. The TPO mimetic for use of any one of claims 1-13, wherein the TPO mimetic is administered to the subject 24 hours before to 24 hours after, preferably about 2 hours to 24 hours before or after, the subject is administered a dose of radiation.

15. The TPO mimetic for use of any one of claims 1-14, wherein the TPO mimetic is administered to the subject by any one of intravenous, intramuscular, intracutaneous, or subcutaneous injection.

16. The TPO mimetic for use of any one of claims 1-15, wherein administration of the TPO mimetic results in at least one of an increased liver capacity of non-irradiated lobe of liver, an increased hypotrophy of irradiated tissue, and a reduced elevation of a circulating liver injury marker, such as hyaluronic acid or a liver transaminase, in blood, in the subject.

17. The TPO mimetic for use of any one of claims 1-16, wherein the TPO mimetic is RWJ 800088 or romiplostim.

## Patentansprüche

1. Thrombopoietin(TPO)-Mimetikum, umfassend die Aminosäuresequenz von SEQ ID NO: 1 zur Verwendung bei einem Verfahren zum Lindern einer durch gezielte Strahlentherapie induzierten Lebererkrankung oder einer durch einen radiomimetischen Wirkstoff induzierten Lebererkrankung bei einem Subjekt, das dessen bedarf.

2. TPO-Mimetikum zur Verwendung nach Anspruch 1, wobei das TPO-Mimetikum in Kombination mit der gezielten Strahlentherapie oder dem radiomimetischen Wirkstoff verabreicht wird.

3. TPO-Mimetikum zur Verwendung nach Anspruch 1 oder 2, wobei die durch gezielte Strahlentherapie induzierte Lebererkrankung aus der Gruppe bestehend aus Hepatomegalie, Lebernekrose, erhöhten Leberenzymen, sinusoidalem Obstruktionssyndrom (SOS) der Leber, Lebervenen- Verschlusskrankheit (VOD) und Leberfibrose ausgewählt ist.

4. TPO-Mimetikum zur Verwendung nach einem der Ansprüche 1-3, wobei das Verfahren (i) eine Leberhypertrophie einer nicht bestrahlten Leber fördert, (ii) Transplantieren von Lebersinus-Endothelzellen (LSEC) und/oder Hepatozyten in bestrahlte Leber fördert und/oder (iii) Leberschädigung entweder allein oder in Kombination mit einer Verabreichung von LSEC verringert.

5. TPO-Mimetikum zur Verwendung nach einem der Ansprüche 1-4, wobei das TPO-Mimetikum dem Subjekt in Kombination mit mindestens einem von Leberzellen und einem Proteinfaktor verabreicht wird, wobei die Leberzellen mindestens eines ausgewählt aus der Gruppe bestehend aus Lebersinus-Endothelzellen (LSEC), Hepatozyten, Leberprogenitorzellen, Leberstammzellen, pluripotenten Stammzellen und rekombinanten Leberzellen, die ein Produkt einer exogenen Polynukleotidsequenz exprimieren, umfassen; die Leberzellen vorzugsweise LSEC und optional Hepatozyten umfassen, und wobei der Proteinfaktor einen Wachstumsfaktor und optional einen Hepatozytenwachstumsfaktor umfasst, wobei der Wachstumsfaktor mehr bevorzugt eines oder mehrere von einem CXCR4-Antagonisten, SDF1, VEGF-A, VEGF-E, FGF-2, EGF, GM-CSF, GCSF, FLT3, Respondin-1, Amphiregulin und MMP14 umfasst.

6. TPO-Mimetikum zur Verwendung nach Anspruch 5, wobei das TPO-Mimetikum dem Subjekt in Kombination mit LSECs, die dem Subjekt vor, nach oder gleichzeitig mit der Verabreichung des TPO-Mimetikums transplantiert werden, verabreicht wird.

7. TPO-Mimetikum zur Verwendung nach Anspruch 4, wobei das TPO-Mimetikum dem Subjekt in Kombination mit LSEC und/oder Hepatozyten verabreicht wird, die dem Subjekt zusammen mit einem Proteinfaktor und optional Hepatozytenwachstumsfaktor verabreicht werden; und ferner der Proteinfaktor mehr bevorzugt ein Stammzellwachstums- und/oderdifferenzierungsfaktor ist, der eines oder mehrere von CXCR4-Antagonist, SDF1, VEGF-A, VEGF-E, FGF-2, EGF, GM-CSF, GCSF, Rspondin-1, FLT3, Amphiregulin und MMP14 umfasst.

8. TPO-Mimetikum zur Verwendung nach Anspruch 7, wobei das TPO-Mimetikum dem Subjekt vor, nach oder gleichzeitig mit einer Transplantation der LSEC und/oder Hepatozyten verabreicht wird.

9. TPO-Mimetikum zur Verwendung nach einem der Ansprüche 1-8, wobei das Subjekt mit einer stereotaktischen Strahlentherapie oder transarteriellen Chemoembolisation (TACE) für die Lebererkrankung behandelt wird.

10. TPO-Mimetikum zur Verwendung nach Anspruch 9, wobei das Subjekt mit einer Dosis von 10-70 Gray (Gy) in 1 bis 10 Fraktionen mit gezielter Bestrahlung behandelt wird.

11. TPO-Mimetikum zur Verwendung nach einem der Ansprüche 1-10, wobei das Subjekt gegen einen Lebertumor oder eine Lebermetastase, vorzugsweise einen Leberkrebs, mehr bevorzugt ein hepatozelluläres Karzinom (HCC), behandelt wird.

12. TPO-Mimetikum zur Verwendung nach einem der Ansprüche 1-10, wobei das Subjekt mit einer präparativen Leberbestrahlung (HIR) zum Engraftment von Leberzellen behandelt wird.

13. TPO-Mimetikum zur Verwendung nach einem der Ansprüche 1-10, wobei das Subjekt mit einer Strahlentherapie für einen gastrointestinalen Krebs behandelt wird oder das Subjekt mit einer präparativen Bestrahlung für eine Knochenmarktransplantation behandelt wird.

14. TPO-Mimetikum zur Verwendung nach einem der Ansprüche 1-13, wobei das TPO-Mimetikum dem Subjekt 24 Stunden bevor bis 24 Stunden nachdem, vorzugsweise etwa 2 Stunden bis 24 Stunden bevor oder nachdem dem Subjekt eine Strahlendosis verabreicht wird, verabreicht wird.

15. TPO-Mimetikum zur Verwendung nach einem der Ansprüche 1-14, wobei das TPO-Mimetikum dem Subjekt durch eine intravenöse, intramuskuläre, intrakutane oder subkutane Injektion verabreicht wird.

16. TPO-Mimetikum zur Verwendung nach einem der Ansprüche 1-15, wobei eine Verabreichung des TPO-Mimetikums bei dem Subjekt zu mindestens einem von einer erhöhten Leberkapazität des nicht bestrahlten Leberlappens, einer erhöhten Hypotrophie des bestrahlten Gewebes und einem verringerten Anstieg eines zirkulierenden Leberverletzungsmarkers, wie etwa Hyaluronsäure oder einer Lebertransaminase, im Blut führt.

17. TPO-Mimetikum zur Verwendung nach einem der Ansprüche 1-16, wobei das TPO-Mimetikum RWJ 800088 oder Romiplostim ist.

## Revendications

1. Mimétique de thrombopoïétine (TPO) comprenant la séquence d'acides aminés SEQ ID NO: 1 destiné à être utilisé dans un procédé d'atténuation d'une maladie hépatique induite par radiothérapie ciblée ou d'une maladie hépatique induite par un agent radiomimétique chez un sujet en ayant besoin.

2. Mimétique de TPO destiné à être utilisé selon la revendication 1, dans lequel le mimétique de TPO est administré en association avec la radiothérapie ciblée ou l'agent radiomimétique.

3. Mimétique de TPO destiné à être utilisé selon la revendication 1 ou 2, dans lequel la maladie hépatique induite par radiothérapie ciblée est choisie dans le groupe constitué de l'hépatomégalie, la nécrose hépatique, l'élévation des enzymes hépatiques, le syndrome d'obstruction sinusoïdale (SOS) hépatique, la maladie occlusive veineuse centrale (VOD) hépatique et la fibrose hépatique.

4. Mimétique de TPO destiné à être utilisé selon l'une quelconque des revendications 1 à 3, dans lequel le procédé (i) favorise l'hypertrophie hépatique du foie non irradié, (ii) favorise la greffe de cellules endothéliales sinusoïdales du foie (LSEC) et/ou d'hépatocytes dans le foie irradié, et/ou (iii) réduit l'endommagement du foie soit seul, soit en combinaison avec l'administration de LSEC.

5. Mimétique de TPO destiné à être utilisé selon l'une quelconque des revendications 1 à 4, dans lequel le mimétique de TPO est administré au sujet en combinaison avec au moins l'un des cellules hépatiques et d'un facteur protéique, dans lequel les cellules hépatiques comprennent au moins l'un choisi parmi le groupe constitué des cellules endothéliales sinusoïdales hépatiques (LSEC), des hépatocytes, des cellules progénitrices hépatiques, des cellules souches hépatiques, des cellules souches pluripotentes, et des cellules hépatiques recombinantes exprimant un produit d'une séquence polynucléotidique exogène ; de préférence les cellules hépatiques comprennent des LSEC, et éventuellement des hépatocytes, et dans lequel le facteur protéique comprend un facteur de croissance et éventuellement un facteur de croissance hépatocytaire, plus préférablement, le facteur de croissance comprend un ou plusieurs parmi un antagoniste de CXCR4, SDF1, VEGF-A, VEGF-E, FGF-2, EGF, GM-CSF, GCSF, FLT3, Rspondin-1, amphiréguline et MMP14.

6. Mimétique de TPO destiné à être utilisé selon la revendication 5, dans lequel le mimétique de TPO est administré au sujet en association avec des LSEC qui lui sont transplantées, avant, après ou simultanément à l'administration du mimétique de TPO.

7. Mimétique de TPO destiné à être utilisé selon la revendication 4, dans lequel le mimétique de TPO est administré au sujet en combinaison avec des LSEC et/ou des hépatocytes, qui sont administrés au sujet conjointement avec un facteur protéique, et éventuellement un facteur de croissance des hépatocytes ; et en outre, plus préférablement, le facteur protéique est un facteur de croissance et/ou de différenciation de cellules souches comprenant l'un ou plusieurs d'un antagoniste de CXCR4, SDF1, VEGF-A, VEGF-E, FGF-2, EGF, GM-CSF, GCSF, Rspondin-1, FLT3, amphiréguline, et MMP14.

8. Mimétique de TPO destiné à être utilisé selon la revendication 7, dans lequel le mimétique de TPO est administré au sujet avant, après ou simultanément à une transplantation des LSEC et/ou des hépatocytes.

9. Mimétique de TPO destiné à être utilisé selon l'une quelconque des revendications 1 à 8, dans lequel le sujet est traité par radiothérapie stéréotaxique ou chimioembolisation transartérielle (TACE) pour la maladie hépatique.

10. Mimétique de TPO destiné à être utilisé selon la revendication 9, dans lequel le sujet est traité par un rayonnement ciblé à une dose de 10-70 Gray (Gy) en 1 à 10 fractions.

11. Mimétique de TPO destiné à être utilisé selon l'une quelconque des revendications 1 à 10, dans lequel le sujet est traité pour une tumeur hépatique ou une métastase hépatique, de préférence un cancer du foie, plus préférablement un carcinome hépatocellulaire (HCC).

12. Mimétique de TPO destiné à être utilisé selon l'une quelconque des revendications 1 à 10, dans lequel le sujet est traité par une irradiation hépatique préparative (HIR) pour la prise de greffe de cellules hépatiques.

13. Mimétique de TPO destiné à être utilisé selon l'une quelconque des revendications 1 à 10, dans lequel le sujet est traité par une radiothérapie pour un cancer gastro-intestinal ou le sujet est traité par une irradiation préparative pour une greffe de moelle osseuse.

14. Mimétique de TPO destiné à être utilisé selon l'une quelconque des revendications 1 à 13, dans lequel le mimétique de TPO est administré au sujet 24 heures avant à 24 heures après, de préférence environ 2 heures à 24 heures avant ou après, une dose de rayonnement étant administrée au sujet.

15. Mimétique de TPO destiné à être utilisé selon l'une quelconque des revendications 1 à 14, dans lequel le mimétique de TPO est administré au sujet par injection intraveineuse, intramusculaire, intracutanée ou sous-cutanée.

16. Mimétique de TPO destiné à être utilisé selon l'une quelconque des revendications 1 à 15, dans lequel l'administration du mimétique de TPO entraîne chez le sujet au moins l'une d'une augmentation de la capacité hépatique du lobe du foie non irradié, une augmentation de l'hypotrophie du tissu irradié et/ou une réduction de l'élévation d'un marqueur de lésion hépatique circulant, tel que l'acide hyaluronique ou une transaminase hépatique, dans le sang.

17. Mimétique de TPO destiné à être utilisé selon l'une quelconque des revendications 1 à 16, dans lequel le mimétique de TPO est RWJ 800088 ou romiplostim.
